# EUROPEAN PATENT APPLICATION

(11) **EP 4 840 966 A1**
(43) Date of publication of application: **18.11.2026**
(21) Application number: 25175905.6
(22) Date of filing: 13.05.2025
(51) Int. Cl.: C12P 21/02, C12N 1/06, C12N 1/08, C12N 1/36, C12P 39/00

(54) **CO-CULTIVATION OF BACTERIAL PRODUCER AND HELPER CELLS**

(71) Applicant: enGenes Biotech GmbH, 1190 Wien (AT)
(72) Inventor: MAIRHOFER, Jürgen, 1190 Wien (AT)
(74) Representative: Schiweck Weinzierl Koch Patentanwälte Partnerschaft mbB

(57) **Abstract**

The present invention is in the field of fermentative biotechnology and, inter alia, relates to methods and uses for the production of a molecule of interest, comprising co-culturing bacterial producer cells and bacterial helper cells, wherein said bacterial producer cells are genetically engineered to produce said molecule of interest, and said bacterial helper cells are genetically engineered to produce a helper protein that is secreted into the extracellular medium, thereby aiding in the production of said molecule of interest as well as to a bacterial consortium comprising bacterial producer cells and bacterial helper cells, as shown for enzymes to be produced and a nuclease as helper to degrade nucleic acid released during lysis of producer cells.

## Description

### I. FIELD OF THE INVENTION

The present invention is in the field of fermentative biotechnology and, inter alia, relates to a method for the production of a molecule of interest, comprising co-culturing bacterial producer cells and bacterial helper cells, wherein said bacterial producer cells are genetically engineered to produce said molecule of interest, and said bacterial helper cells are genetically engineered to produce a helper protein that is secreted into the extracellular medium, thereby aiding in the production of said molecule of interest. The present invention further relates to the use of co-cultured bacterial producer cells and bacterial helper cells, wherein said bacterial producer cells and/or bacterial helper cells are growth and production decoupled, wherein said bacterial producer cells are genetically-engineered to produce said molecule of interest, and said bacterial helper cells are genetically-engineered to produce a helper protein aiding in the production of said molecule of interest, for the production of a molecule of interest. Furthermore, the present invention relates to a bacterial consortium comprising bacterial producer cells which are genetically-engineered to produce a molecule of interest and bacterial helper cells which are genetically-engineered to produce a protein aiding in the production of said molecule of interest, wherein each of said bacterial producer cells and bacterial helper cells is equipped such that growth and production can be decoupled.

### II. BACKGROUND

Production of molecules of interest (MOI), e.g., proteins of interest (POI) or nucleotides of interest, has been accomplished with many prokaryotic hosts. The most prominent examples are bacteria like *Escherichia coli, Bacillus subtilis, Pseudomonas fluorescens, Streptomyces griseus,* or *Corynebacterium glutamicum.* A great number of biological pharmaceuticals (e.g. antibodies or functional fragments thereof) have been produced in the last decade and an increasing number is nearing approval for use in humans but their efficient production remains a challenging task. Therapeutically active doses are often in the order of milligram (mg) per administration. Thus, considerable amounts of protein are needed as active ingredients, making an efficient and cost-effective production worthwhile.

Bacterial cell expression systems have long been, and still are, one of the major tools for production of these types of molecules. The key objective of process optimization is to achieve a high yield of product having the required quality at the lowest possible cost, which is often determined by the properties of a specific expression construct or system. For example, high-level recombinant protein expression may overwhelm the metabolic capacity of a host cell and consequently leads to plasmid loss, reduced oxygen uptake, generation of toxic by-products, formation of inclusion bodies, and/or triggering of a stress response which often impairs efficient protein production. It is also known that sometimes high expression of an mRNA encoding a protein of interest does not necessarily lead to high amounts of the protein. Different approaches have been taken by scientists to deal with these problems. As another example, the expression of a recombinant protein can be further increased by optimizing the gene dosage encoding the protein of interest, by using a suitable promoter or by optimizing the codon usage of the gene encoding the protein of interest according to the employed host cell. Several other parameters have been shown to affect the expression level of a recombinant protein in a host cell, such as expression vector design, media composition, growth temperature, chaperone co-expression, mRNA stability, translation initiation and epigenetic processes. However, high level of protein yield in host cells may be limited at one or more different steps, like folding, disulfide bond formation, transport within the cell, or release from the cell. Also lysis of host cells is an issue (Berg (2023), Separation and Purification Technology 321, 124540 (doi.org/10.1016/j.seppur.2023.124540) .

The prior art tackled the latter issue by co-expressing in one and the same bacterial host cell a protein of interest and helper protein, e.g. a Fab fragment and a nuclease. The nuclease should help in degrading DNA released from broken bacterial cells when the Fab fragment is harvested. This approach was made to facilitate downstream processing, because DNA released from broken cells renders liquid solution extremely viscous, thereby hampering downstream processes; Balasundaram (2009), Biotechnology Bioengineering 104 (): 134-142. However, titres of the protein of interest were much lower than expressing a protein of interest traditionally, i.e., without co-expression of a helper protein, e.g. a nuclease.

Accordingly, in another approach, a protein of interest is traditionally expressed in a bacterial cell and exogenous enzymes, e.g. a nuclease, such as benzoanse is added prior to or at the time when downstream processing is started in order to degrade unwanted by-products, e.g. DNA released from broken bacterial cells which would otherwise hamper downstream processing due to rendering liquid solutions viscous; see Berg (2023), cited above.. However, this approach is quite costly and not always satisfyingly efficient.

These and further disadvantages need to be overcome. The present invention therefore addresses these needs and technical objectives and provides a solution as described herein, as defined in the claims, and illustrated in the Examples.

Accordingly, one technical problem underlying the present invention is to offer an efficient or more efficient method for the production of a molecule of interest.

### III. SUMMARY OF THE INVENTION

The present invention provides as a solution to the technical problem methods and uses to aid in the (more) efficient production of a molecule of interest.

The present invention provides a method for production of a molecule of interest by co-culturing bacterial producer cells and bacterial helper cells, wherein said bacterial producer cells are able (e.g., genetically engineered) to produce said molecule of interest, and said bacterial helper cells are able (e.g., genetically engineered) to produce a helper protein that is secreted into the extracellular medium.

By way of co-culturing bacterial producer and helper cells, the present invention addresses simultaneously at least two needs set out in the prior art, i.e., no exogenous addition of a helper protein is needed and higher tires are achieved in comparison to co-expressing a molecule of interest, e.g. a protein of interest and a helper protein in one and the same bacterial cell.

Accordingly, the present invention relates to a method for the production of a molecule of interest, comprising co-culturing bacterial producer cells and bacterial helper cells, wherein said bacterial producer cells are genetically engineered to produce said molecule of interest, and said bacterial helper cells are genetically engineered to produce a helper protein that is secreted into the extracellular medium, thereby aiding in the production of said molecule of interest.

In one embodiment of the invention, the method further comprising providing a preparation of said bacterial producer cells and/or said bacterial helper cells.

In one embodiment of the invention, the method comprises decoupling growth and/or production of said bacterial producer cells and said bacterial helper cells. In a more specific embodiment of the present invention, growth and production of both, bacterial producer cells and bacterial helper cells are decoupled. In another more specific embodiment of the present invention, only growth and production of the bacterial producer cells is decoupled. In yet another more specific embodiment of the present invention, only growth and production of the bacterial helper cells is decoupled.

In one embodiment of the invention, the molecule of interest produced by said bacterial producer cells, the molecule of interest produced by said bacterial producer cells is a protein causing cell lysis in said bacterial producer cells when said molecule of interest is produced by said bacterial producer cells.

In one embodiment of the invention, the molecule of interest produced by said bacterial producer cells is an enzyme or antibody. In a more specific embodiment of the invention, said enzyme is a polyethylene terephthalate hydrolase (PETase, e.g. a PETase according to EC 3.1.1.101), or a protease.

In one embodiment of the invention, the helper protein expressed by said bacterial helper cells reduces an impurity caused by said bacterial producer cells. In a more specific embodiment of the invention, said impurity is a nucleic acid, e.g., a DNA or RNA. In one specific embodiment of the invention, said DNA is bacterial chromosomal DNA, mitochondrial DNA, or extrachromosomal DNA.

In one embodiment of the invention, particularly where said helper protein expressed by said bacterial helper cells reduces an impurity caused by said bacterial producer cells, said helper protein is a nuclease. In a more specific embodiment of the invention, said nuclease is nuclease A from *Serratia marcescens.*

In another embodiment of the invention, the helper protein expressed by said bacterial helper cells reduces an impurity caused by said bacterial producer cells. In a more specific embodiment of the invention, said impurity is lipopolysaccharide (LPS).

In one embodiment of the invention, particularly where said helper protein expressed by said bacterial helper cells reduces an impurity caused by said bacterial producer cells, wherein said impurity is lipopolysaccharide, said helper protein is lipopolysaccharide binding protein, preferably a phage tail spike protein (TSP). Such TSP is capable of binding lipopolysaccharide.

Preferably, the phage tail spike protein (TSP) is T4-like bacteriophage gp12, T4 phage long tail fiber protein gp37, bacteriophage Bp7 receptor-recognizing protein gp38, E. coli phage DT571/2 tail fiber protein LtfA, E. coli phage DT571/2 tail fiber protein B, E. coli phage P2 tail fiber assembly protein G, E. coli phage Mu tail fiber assembly protein U', E34 phage TSP, Salmonella phage P22 TSP.

In another embodiment of the invention, particularly where said helper protein expressed by said bacterial helper cells reduces an impurity caused by said bacterial producer cells, wherein said impurity is lipopolysaccharide, said helper protein is a lipopolysaccharide degrading protein.

Preferably, said lipopolysaccharide degrading protein is Cobetia amphilecti alkaline phosphatase, calf intestinal alkaline phosphatase, macrophage acyloxyacyl hydrolase (AOAH), polysaccharide lyase, e.g. from the phage LKA1 tailspike, or the lipopolysaccharide export system protein LptA from E. coli.

In one embodiment of the invention, said helper protein is secreted by said bacterial helper cells.

In one embodiment of the invention, said molecule of interest produced by said bacterial producer cells is a pre-protein. In a more specific embodiment of the invention, said helper protein expressed by said bacterial helper cells processes said pre-protein produced by said bacterial producer cells.

In one embodiment of the invention, particularly where the molecule of interest produced by said bacterial producer cells is a pre-protein and said helper protein expressed by said bacterial helper cells processes said pre-protein produced by said bacterial producer cells, said helper protein is a protease. In a more specific embodiment of the present invention, said protease is an endopeptidase and/or exopeptidase. In a more specific embodiment of the present invention, said protease is an aspartate protease, cysteine protease, glutamate protease, metalloprotease, serine protease, threonine protease or methionine aminopeptidase.

In one embodiment of the invention, said molecule of interest produced by said bacterial producer cells is an antibody.

In one embodiment of the invention, said helper protein expressed by said bacterial helper cells binds said antibody produced by said bacterial producer cells.

In one embodiment of the invention, particularly where said molecule of interest produced by said bacterial producer cells is an antibody and where helper protein expressed by said bacterial helper cells binds said antibody produced by said bacterial producer cells, said helper protein is protein A, G or L.

In one embodiment of the invention, said bacterial producer cells and/or bacterial helper cells are equipped such that growth and production can be decoupled.

In one embodiment of the invention, said bacterial producer cells and/or bacterial helper cells are genetically engineered with a construct which inhibits growth of said bacterial cells.

In one embodiment of the invention, each of said bacterial producer cells and said bacterial helper cells is cultivated in a separate pre-culture.

In one embodiment of the invention, said method as described herein comprises a step wherein, after separate pre-culturing, said bacterial producer cells and said bacterial helper cells are mixed in a pre-defined ratio for being co-cultured.

The invention further relates to the use of co-cultured bacterial producer cells and bacterial helper cells, wherein said bacterial producer cells and/or bacterial helper cells are growth and production decoupled, wherein said bacterial producer cells are genetically engineered to produce said molecule of interest, and said bacterial helper cells are genetically engineered to produce a helper protein aiding in the production of said molecule of interest, for the production of a molecule of interest.

The invention further relates to a bacterial consortium comprising bacterial producer cells which are genetically-engineered to produce a molecule of interest and bacterial helper cells which are genetically-engineered to produce a protein aiding in the production of said molecule of interest, wherein each of said bacterial producer cells and bacterial helper cells is equipped such that growth and production can be decoupled.

In one embodiment of the present invention, said bacterial consortium, each of said bacterial producer cells and bacterial helper cells is genetically engineered with a construct which inhibits growth of said bacterial cells.

In a more specific embodiment of the present invention, said bacterial producer cells of said bacterial consortium produce an enzyme and said bacterial helper cells of said bacterial consortium produce a nuclease.

In another embodiment of the present invention, said bacterial producer cells of said bacterial consortium produce a pre-protein and said bacterial helper cells of said bacterial consortium produce a protease.

In another embodiment of the present invention, said bacterial producer cells of said bacterial consortium produce an antibody and said bacterial helper cells of said bacterial consortium produce protein A, G or L.

### IV. BRIEF DESCRIPTION OF THE DRAWINGS

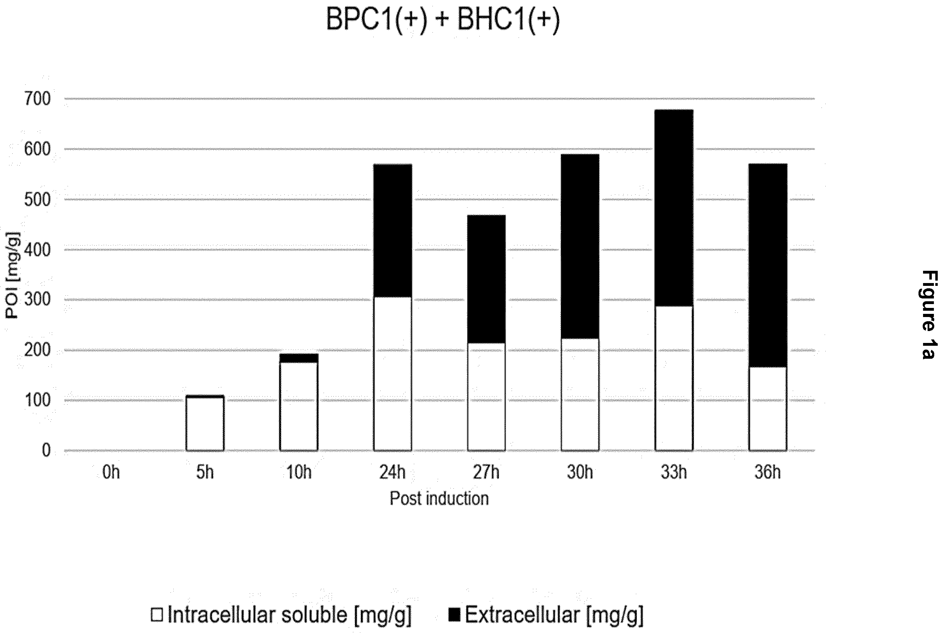

**Figure 1a** **to d:** each of Figures a to d shows specific titres (mg molecule of interest per gram cell dry mass) of different co-culture experiments of bacterial producer cells and helper cells as described in the present application over 36 hours, where **(a)** both bacterial and helper cells are decoupled in growth and production; **(b)** bacterial producer cells are decoupled in growth and production, while bacterial helper cells are not; (c) bacterial helper cells are not decoupled in growth and production, while bacterial helper cells are; or **(d)** neither bacterial producer cells nor bacterial helper cells are decoupled in growth and production.

Bars show the specific titre at certain time points. The filled area of the bar shows the amount of the specific extracellular titre, while the non-filled area of the bar shows the amount of the specific intracellular titre.

BPC1 stands for a bacterial producer cell which expresses a molecule of interest, here an endopeptidase.

BHC1 stands for a bacterial helper which expresses a helper protein, here a nuclease.

"+" (in words: plus) stands for cells decoupled in growth and production.

"-" (in words: minus) stands for cells which are not decoupled in growth and production.

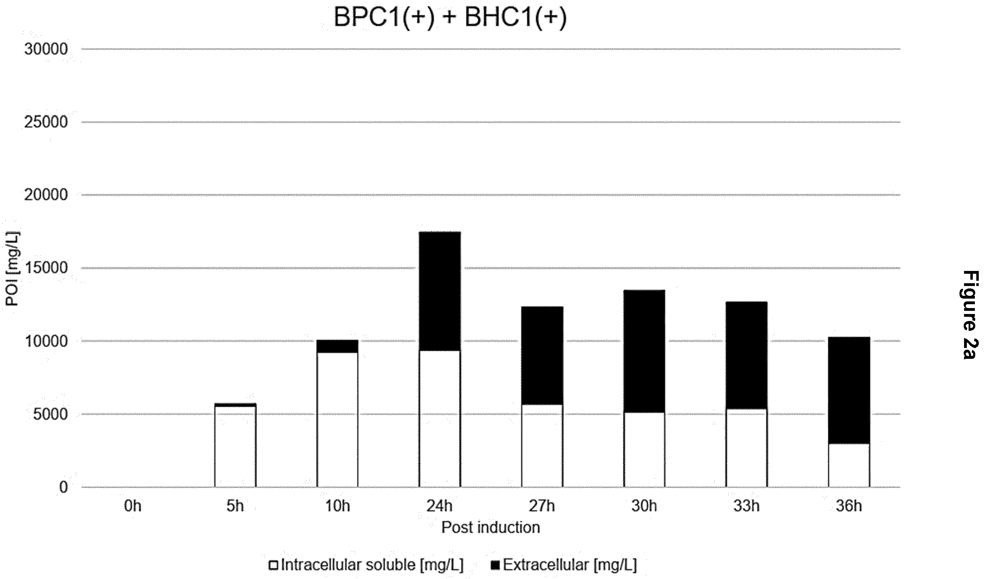

**Figure 2a** **to d:** each Figures a to d shows volumetric titres (mg molecule of interest per litre cell culture) of different co-culture experiments of bacterial producer cells and helper cells as described in the present application over 36 hours, where **(a)** both bacterial and helper cells are decoupled in growth and production; **(b)** bacterial producer cells are decoupled in growth and production, while bacterial helper cells are not; **(c)** bacterial helper cells are not decoupled in growth and production, while bacterial helper cells are; or **(d)** neither bacterial producer cells nor bacterial helper cells are decoupled in growth and production.

Bars show the specific titre at certain time points. The filled area of the bar shows the amount of the specific extracellular titre, while the non-filled area of the bar shows the amount of the specific intracellular titre.

BPC1 stands for a bacterial producer cell which expresses a molecule of interest, here an endopeptidase.

BHC1 stands for a bacterial helper cell which expresses a helper protein, here a nuclease.

"(+)" (in words: plus in parenthesis) stands for cells decoupled in growth and production.

"(-)" (in words: minus in parenthesis) stands for cells which are not decoupled in growth and production.

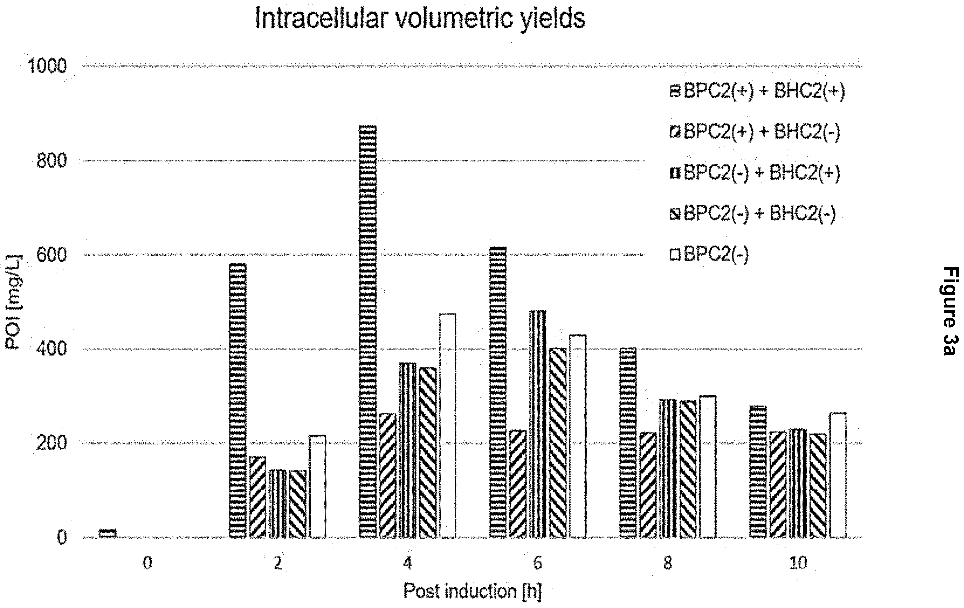

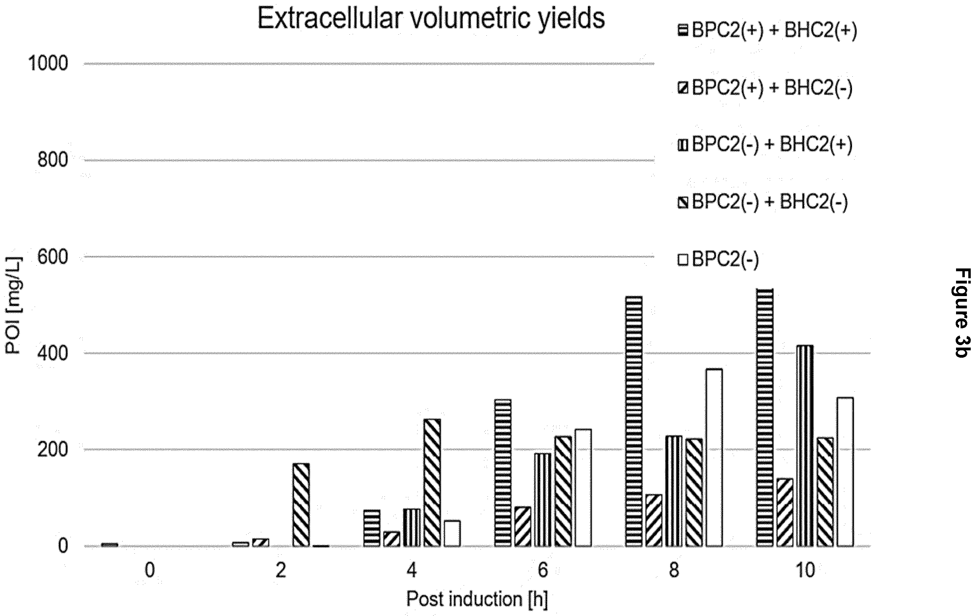

**Figure 3a** **and** **3b****:** each Figure shows intracellular and extracellular volumetric titres, respectively, of different co-culture experiments of bacterial producer cells and helper cells as described in the present application over 10 hours and a single culture experiment of bacterial producer cells.

Co-cultures are (from left to right bar): both bacterial and helper cells are decoupled in growth and production; bacterial producer cells are decoupled in growth and production, while bacterial helper cells are not; bacterial helper cells are not decoupled in growth and production, while bacterial helper cells are; neither bacterial producer cells nor bacterial helper cells are decoupled in growth and production; and bacterial producer cells only which are not decoupled in growth and production.

Bars show the intracellular and extracellular volumetric titre at certain time points, respectively.

BPC2 stands for a bacterial producer which expresses a molecule of interest, here a PETase.

BHC2 stands for a bacterial helper cell which expresses a helper protein, here a nuclease.

"(+)" (in words: plus in parenthesis) stands for cells de-coupled in growth and production.

"(-)" (in words: minus in parenthesis) stands for cells which are not de-coupled in growth and production.

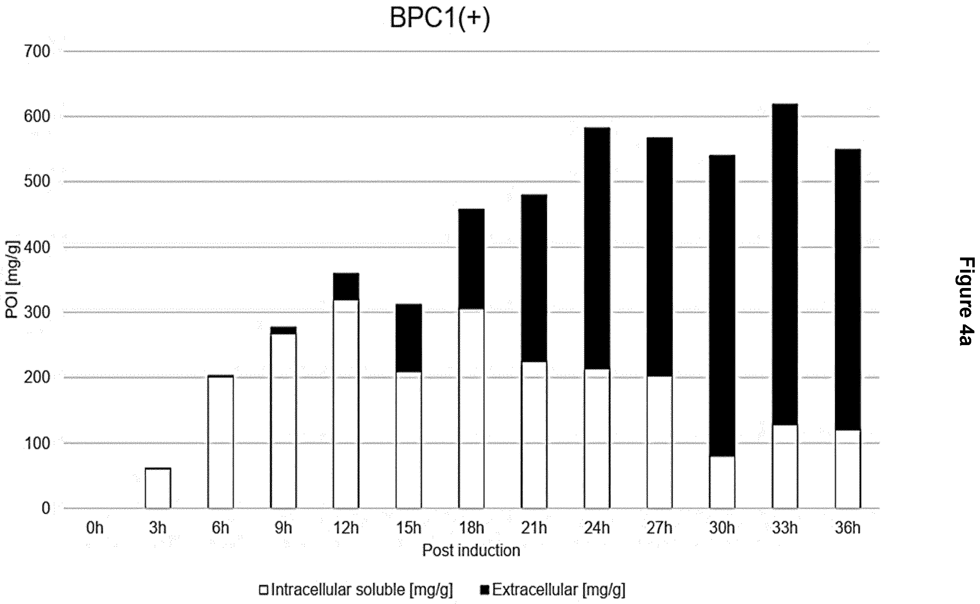

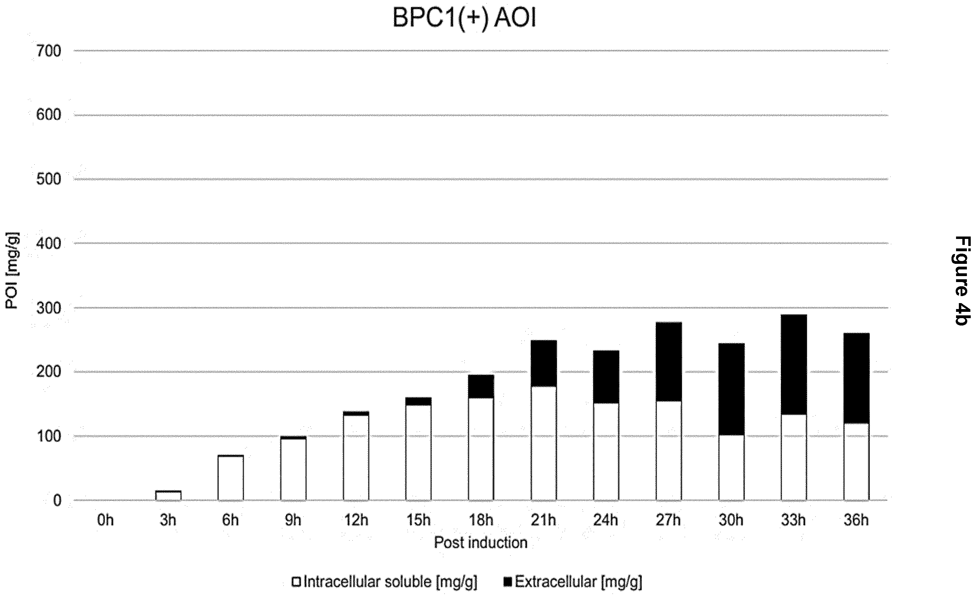

**Figures 4a** **and** **4b****:** each Figure shows specific titres of a single expression and co-expression experiment, respectively, of bacterial producer cells over 36 hours. Bars show the specific titre at certain time points. The filled area of the bar shows the amount of the specific extracellular titre, while the non-filled area of the bar shows the amount of the specific intracellular titre.

BPC1 stands for a bacterial producer cell which expresses a molecule of interest, here an endopeptidase.

BPC (+) AOI stands for a bacterial producer cell which co-expresses a molecule of interest, here an endopeptidase and a helper protein, here a nuclease.

"+" (in words: plus) stands for cells de-coupled in growth and production.

Data and results shown in Figures 4a and 4b are comparative data and results only which are not part of the present invention, but merely serve for the purpose of comparing the concept of the present invention, i.e., co-culture of bacterial producer cells producing a molecule of interest, e.g. a protein causing cell lysis in said bacterial producer cells when said molecule of interest is produced by said bacterial producer cells and bacterial helper cells producing a helper protein, e.g. a nuclease with co-expression of said molecule of interest and said helper protein in one and the same cell as described in the prior art.

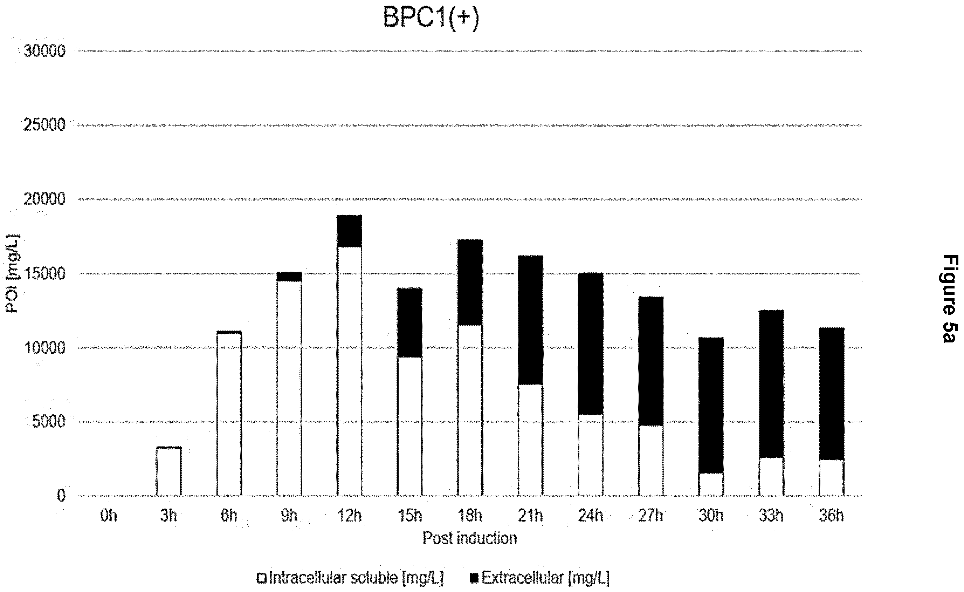

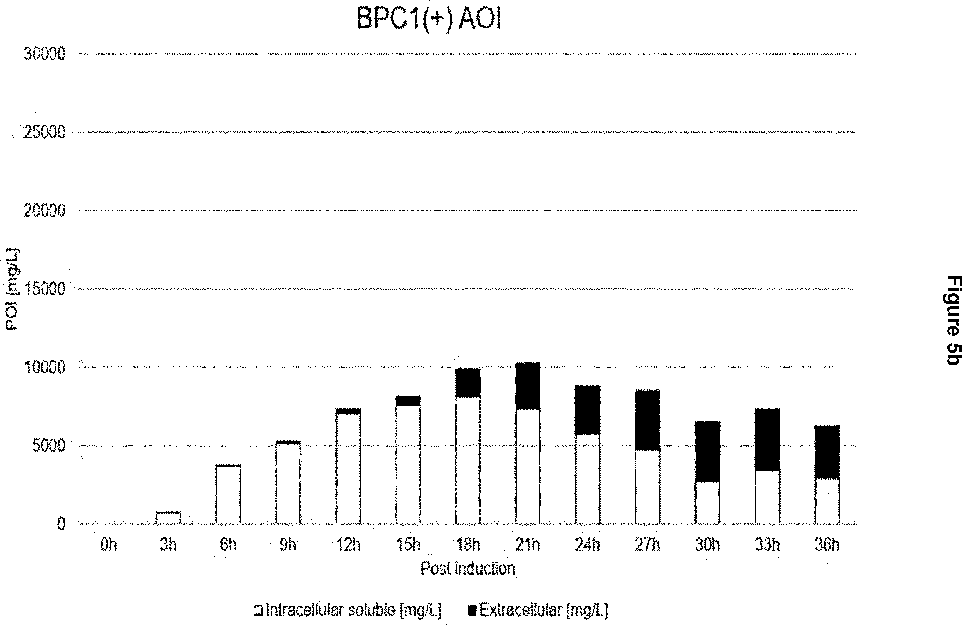

**Figures 5a** **and** **5b****:** each Figure shows volumetric titres of a single expression and co-expression experiment, respectively, over 36 hours. Bars show the volumetric titre at certain time points. The filled area of the bar shows the amount of the volumetric extracellular titre, while the non-filled area of the bar shows the amount of the volumetric intracellular titre.

BPC1 stands for a bacterial producer cell which expresses an endopeptidase.

BPC (+) AOI stands for a bacterial helper cell which co-expresses an endopeptidase and a nuclease.

"+" (in words: plus) stands for cells de-coupled in growth and production.

Data and results shown in Figures 5a and 5b are comparative data and results only which are not part of the present invention, but merely serve for the purpose of comparing the concept of the present invention, i.e., co-culture of bacterial producer cells producing a molecule of interest, e.g. a protein causing cell lysis in said bacterial producer cells when said molecule of interest is produced by said bacterial producer cells and bacterial helper cells producing a helper protein, e.g. a nuclease with co-expression of said molecule of interest and said helper protein in one and the same cell as described in the prior art.

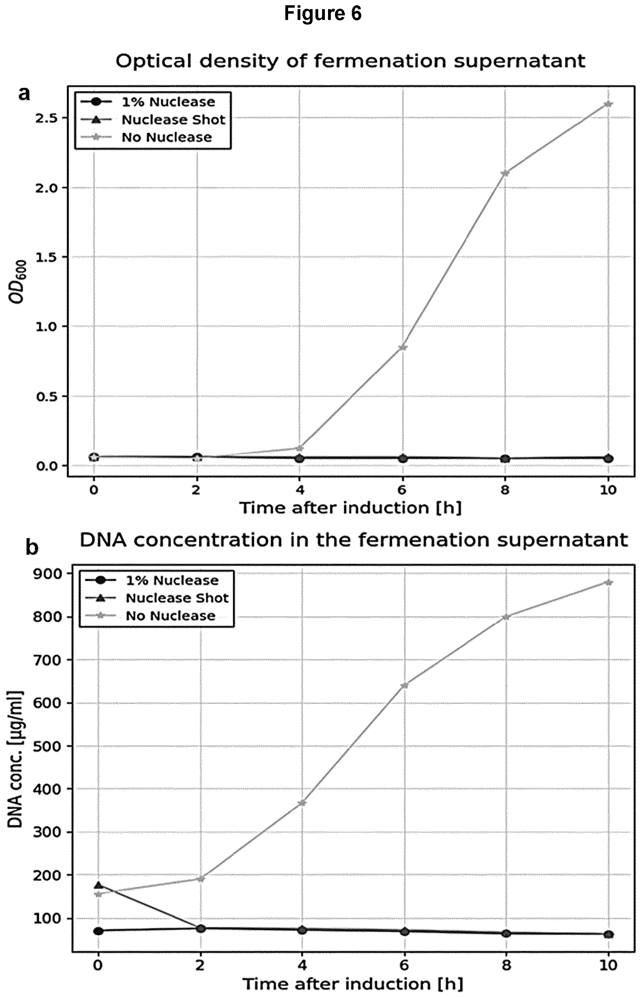

**Figures 6a** **and b: a)** shows optical density at 600 nm (OD600) of the fermentation supernatant at certain points in time of bacterial cells expressing a molecule of interest, here a PETase over 10 hours with or without addition or presence of a helper protein, here a nuclease into the culture medium.

**b)** shows DNA concentration the fermentation supernatant at certain points in time of bacterial cells expressing a molecule of interest, here a PETase over 10 hours with or without addition or presence of a helper protein, here a nuclease into the culture medium.

Curve marked by filled circles shows results of the condition where nuclease is permanently present in the culture medium due to co-culture of bacterial producer cells producing a molecule of interest, here a PETase and bacterial helper cells producing a helper protein, here a nuclease during fermentation. At the start of co-culture bacterial helper cells are added in an amount of 1% relative to the OD600 of bacterial producer cells added at the start of co-culture, e.g. if 10 OD600 of bacterial producer cells is added at the start of cocultivation, then 0.1 OD600 of bacterial helper cells is added at said start.

Curve marked by filled triangles shows a single time addition of nuclease into the culture medium during fermentation.

Curve marked by filled asterisks shows no addition of nuclease into the culture medium during fermentation.

The following remarkable results are apparent from the above-described Figures and provide for advantages of the present invention in view of the prior art.

Remarkably, co-culture of bacterial producer cells producing a molecule of interest and bacterial helper cells producing a helper protein is advantageous over co-expression of said molecule of interest and said helper protein in one and the same bacterial cells.

Figure 1a to d show that the concept of the present invention, i.e., co-culture of bacterial producer cells producing a molecule of interest, e.g. a protein causing cell lysis, e.g. an endopeptidase, in said bacterial producer cells when said molecule of interest is produced by said bacterial producer cells and bacterial helper cells producing a helper protein, e.g. a nuclease, which is secreted into the extracellular medium - here culture medium - is advantageous with respect to the specific titre over the concept of co-expression of said molecule of interest and said helper protein in one and the same cells; see Figure 4b.

Thus, from Figures 1a to d and Figure 4b, it is apparent that co-culture of bacterial producer cells producing a molecule of interest and bacterial helper cells producing a helper protein secreted into the extracellular medium and which aids in the production of said molecule of interest is superior to co-expression of said molecule of interest and said helper protein in one and the same bacterial cell as regards the specific titre.

Figure 2a to d show that the concept of the present invention, i.e., co-culture of bacterial producer cells producing a molecule of interest, e.g. a protein causing cell lysis, e.g. an endopeptidase, in said bacterial producer cells when said molecule of interest is produced by said bacterial producer cells and bacterial helper cells producing a helper protein, e.g. a nuclease, which is secreted into the extracellular medium - here culture medium - is advantageous with respect to the volumetric titre over the concept of co-expression of said molecule of interest and said helper protein in one and the same cells; see Figure 5b.

Thus, from Figures 2a to d and Figure 5b, it is apparent that co-culture of bacterial host cells producing a molecule of interest and bacterial producer cells producing a helper protein secreted into the extracellular medium and which aids in the production of said molecule of interest is superior to co-expression of said molecule of interest and said helper protein in one and the same bacterial cell as regards the volumetric titre.

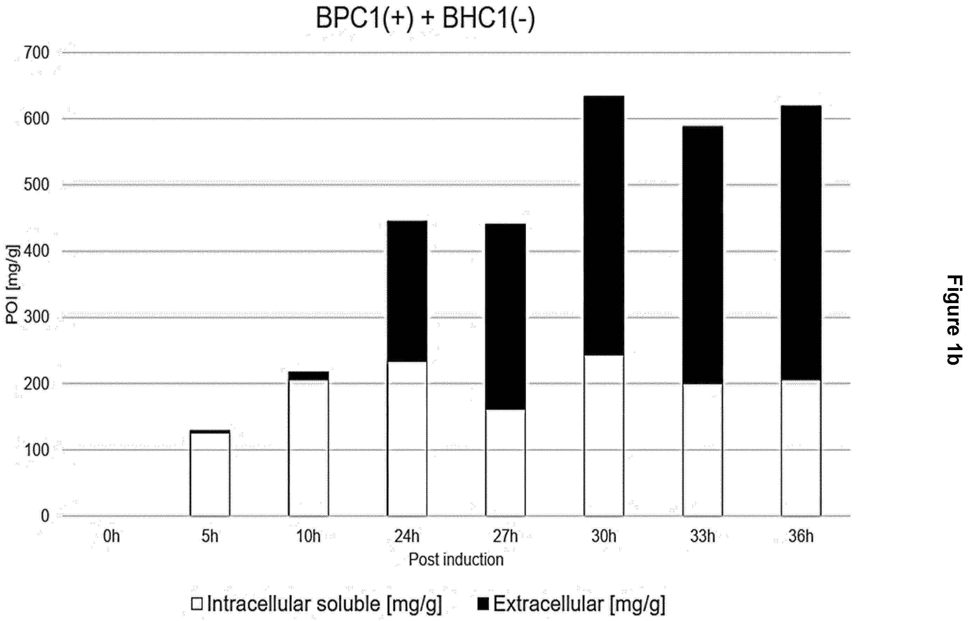

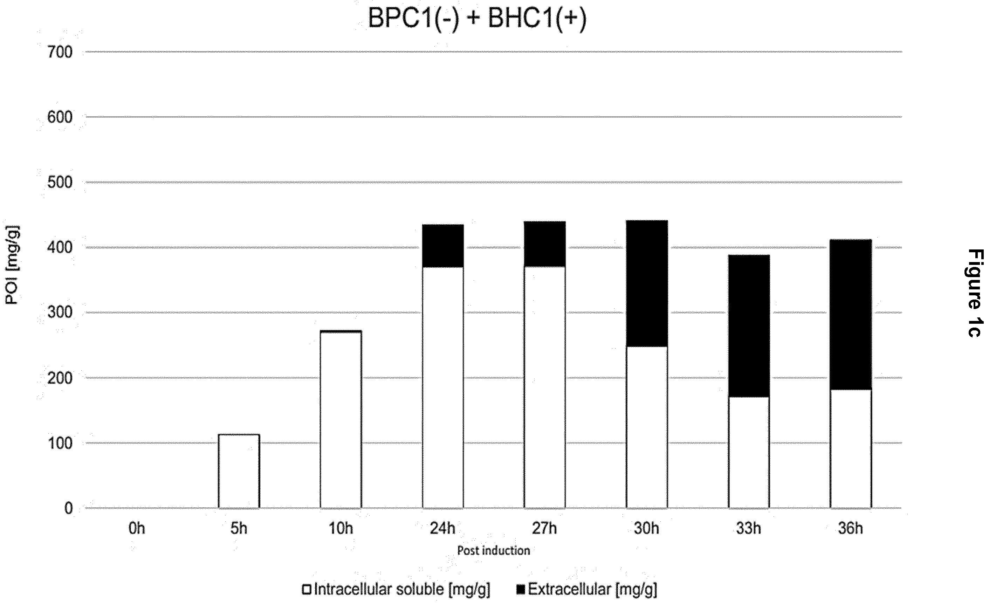

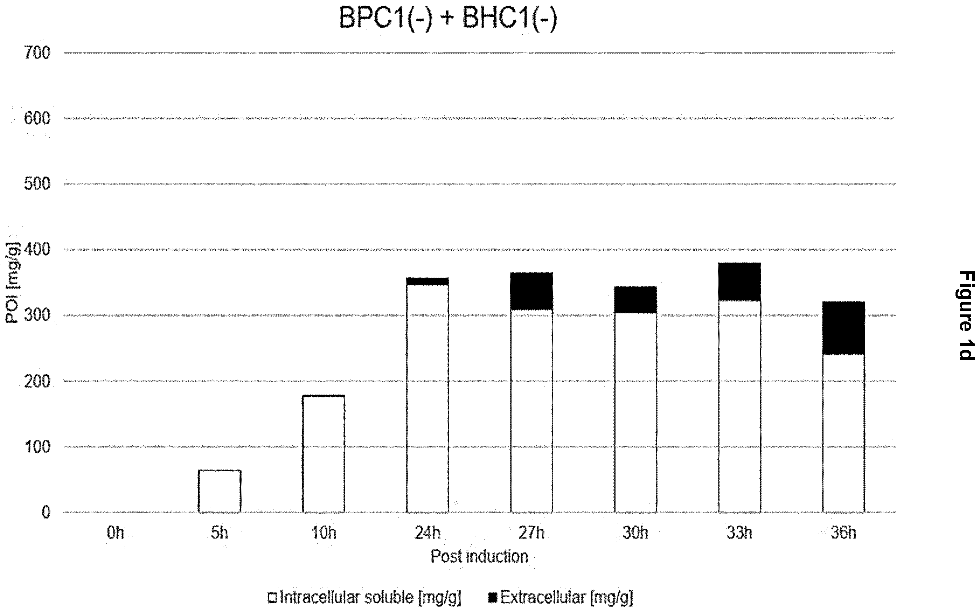

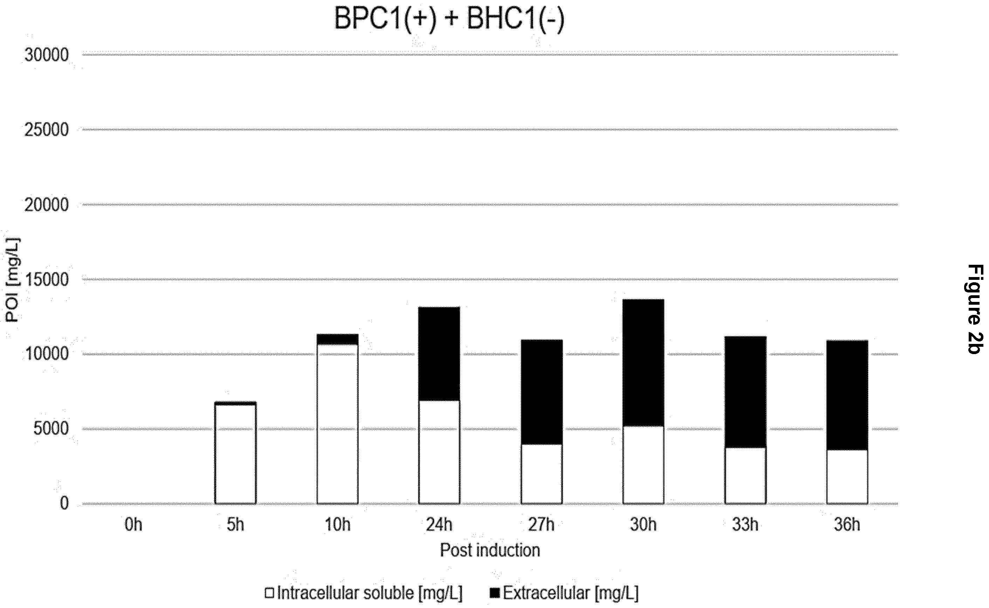

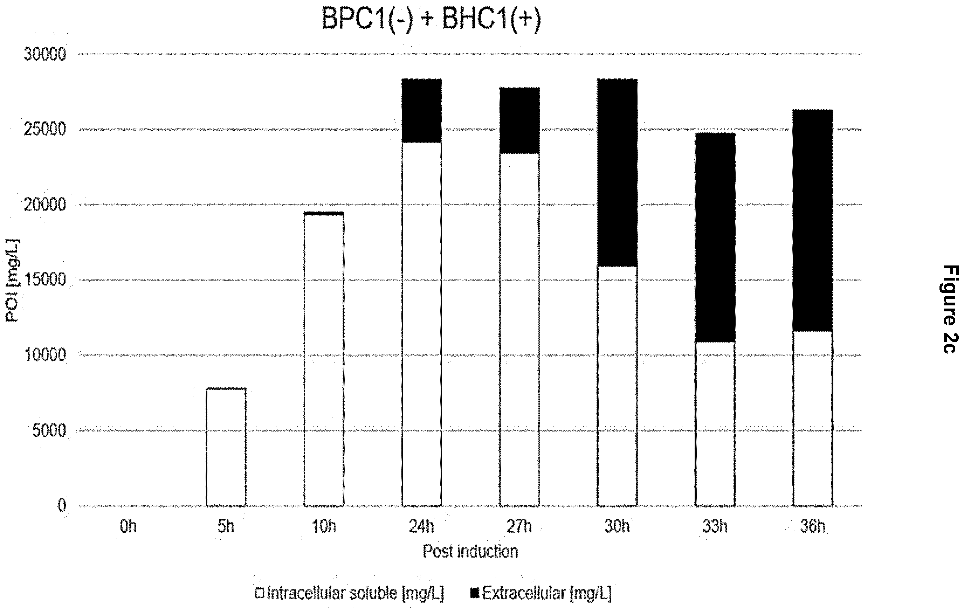

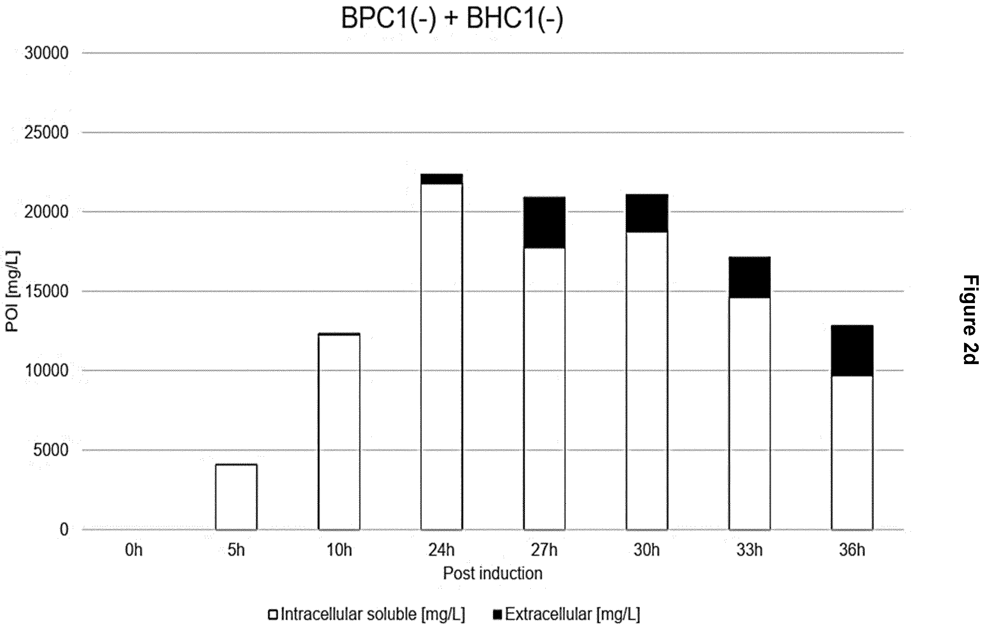

Figures 3a and b provide for the same advantageous results as shown in Figures 1 and 2, with the exception that another molecule of interest, here a PETase is produced by said bacterial producer cells.

Remarkably, co-culture of bacterial producer cells producing a molecule of interest and bacterial helper cells producing a helper protein aiding in the production of said molecule of interest is advantageous over the production of the molecule of interest in the absence of such bacterial helper cells.

When comparing the results shown in Figures 1a to d, Figures 2a to d and Figures 3a and b, to the results shown in Figure 6, it is apparent that co-culture of bacterial producer and helper cells as described herein is advantageous in the production of a molecule of interest, e.g. a protein causing cell lysis, e.g. an endopeptidase, in bacterial producer cells when said molecule of interest is produced by said bacterial producer cells, since bacterial helper cells producing a helper protein which aids in the production of said molecule of interest, e.g. a nuclease, which is secreted into the extracellular medium - here culture medium - degrades nucleic acids, e.g. dsDNA released from lysed bacterial producer cells. The advantage resides in the fact that otherwise released nucleic acids increase viscosity of the culture medium due to free nucleic acids, particularly dsDNA released from said lysed bacterial producer cells such that basically no downstream processing, e.g. harvesting bacterial producer cells or harvesting and/or purifying the molecule of interest is possible.

This is apparent from Figures 6a and b; see curve marked with filled circles ("no nuclease"). Indeed, bacterial producer cells which are lysed when expressing a protein which causes cell lysis when produced in said bacterial producer cells, release in particular dsDNA which increases viscosity of the culture medium such that basically no downstream processing, e.g. harvesting bacterial producer cells or harvesting and/or purifying the molecule of interest is possible.

Figures 6a and b also attest to the fact that a nuclease present in or added to the culture medium aids in the production of a molecule of interest, e.g. a protein causing cell lysis when produced in said bacterial producer cells insofar as a nuclease degrades in particular dsDNA release from lysed bacterial producer cells.

However, rather than adding a helper protein exogenously, the present invention provides for an "in line" helper protein due to co-culturing bacterial producer cells and bacterial helper cells such that the helper protein produced by sad bacterial helper cells aids in the production of the molecule of interest produced by said bacterial producer cells. It is both more effective and cheaper to produce such a helper protein "in line" rather than adding it, perhaps in large amounts, exogenously. Hence, this is a further advantage of the concept of co-culture of bacterial producer and helper cells as described in the context of the present invention.

Also, as demonstrated herein, co-culture of bacterial producer cells producing a molecule of interest and bacterial helper cells producing a helper protein secreted into the extracellular medium is advantageous with respect to both the specific and/or volumetric titre over co-expression of said molecule of interest and said helper protein in one and the same bacterial cell (compare Figures 1a to d to Figure 4b and Figures 2a to d to Figure 5b).

Remarkably, decoupling growth and production is superior to not decoupling growth and production as regards the specific titre of a molecule of interest.

From Figures 1 to d it is also apparent that decoupling either the bacterial producer cells or bacterial helper cells or both fin growth and production, higher specific titres can be achieved (compare Figure 1a to c with 1d).

Remarkably, as is apparent from Figure 6, optical density increases drastically as of 4 hours of fermentation when no nuclease is added which is apparent from very turbid supernatants even after centrifugation.

However, optical density stays constant over the course of fermentation when a nuclease is added or present due to co-cultivated bacterial helper cells pursuant to the concept of the present invention, i.e., co-cultivation of bacterial producer and helper cells.

Thus, it is apparent, that fermentation without nuclease shows high DNA concentration in the supernatant due to lysis of bacterial producer cells when expressing a molecule of interest which causes cell lysis when produced by said bacterial producer cells.

Fermentation with added or co-cultivated nuclease show no increase of DNA throughout the entire production phase. Indeed, 1% (OD units) of the nuclease strain (in relation to the OD of the producer strain) in inoculum produces enough nucleases for the whole process.

Thus, in sum, the present invention provides for the following advantages:
Co-culture of bacterial producer cells producing a molecule of interest and bacterial helper cells producing a helper protein which is secreted into the extracellular medium is advantageous over co-expression of said molecule of interest and said helper protein in one and the same bacterial cells as regards the specific and/or volumetric titre of said molecule of interest.

Co-culture of bacterial producer cells producing a molecule of interest and bacterial helper cells producing a helper protein aiding in the production of said molecule of interest is advantageous over the production of the molecule of interest in the absence of such bacterial helper cells producing a helper protein which is secreted into the extracellular medium, since presence of the helper protein facilitates production of said molecule of interest and/or provides for efficient downstream processing of said molecule of interest and thus helps reducing production costs in comparison to the exogenous addition of such a helper protein during fermentation and/or downstream processing.

Decoupling growth and production of either or both of bacterial producer and bacterial helper cells is superior to not decoupling growth and production as regards the specific titre of a molecule of interest.

### V. DETAILED DESCRIPTION

The present invention provides a method for the production of molecules of interest, comprising the co-culturing of bacterial producer cells (also referred to herein as bacterial host cells) and bacterial helper cells. As described herein, these cells may be genetically engineered to produce a molecule of interest (e.g., a protein or part thereof, for example an enzyme or an antibody or parts thereof) and/or a helper protein, respectively, wherein the helper protein aids the production of the molecule of interest.

A "bacterial producer cell" or sometimes abbreviated as "producer cell" as referred to herein is a bacterial cell which produces a molecule of interest, preferably a molecule of interest as described herein. Accordingly, a bacterial producer cell is transformed with a nucleotide sequence encoding a molecule of interest. The nucleotide sequence of interest may be integrated into the genome of the bacterial producer cell or may be on an extrachromosomal element, such as a plasmid, cosmid or bacmid. A plasmid may be a low, medium or high copy number plasmid. Expression of the nucleotide sequence encoding the molecule of interest may be effected by a promoter which may be constitutive or inducible. It is within the scope of the term "bacterial producer cell" that it may be one, two, three, four, five, six or more bacterial producer cells, each of which produces a different molecule of interest.

A "bacterial helper cell" or sometimes abbreviated as "helper cell" as referred to herein is a bacterial cell which produces a helper protein that is secreted into the extracellular medium.

The bacterial helper cell by producing the helper protein aids in production of said molecule of interest. Accordingly, it is the bacterial helper cell or the helper protein, respectively, which aids in production of said molecule of interest. The helper protein may, as described herein, facilitate the production of a molecule of interest, e.g. it may reduce impurities caused by the production of the molecule of interest by the bacterial producer cell, e.g. it may reduce nucleic acids in the culture medium released, e.g. due to lysis by the bacterial producer cell. Such a reduction of nucleic acids may be achieved by a nuclease. Such impurities may hamper the production of the molecule of interest.

However, the helper protein may not only reduce impurities, it may also aid in the production of the molecule of interest, e.g. by processing said molecule of interest, e.g. by processing a pre-protein, e.g. as described herein, or by binding said molecule of interest, e.g. as described herein, thereby facilitating its isolation.

The production of the molecule of interest includes upstream and downstream processes. The upstream process includes the production of the molecule of interest. The downstream process includes harvesting, isolating and/or purifying the molecule of interest. As described herein, the helper protein referred to herein ads in the production of the molecule of interest.

It is within the scope of the term "bacterial helper cell" that it may be one, two, three, four, five, six or more bacterial helper cells, each of which produces a different helper protein.

"Titre" when used herein refers - dependent on the context - to either the "specific titre" or the "volumetric titre".

The "specific titre" is the amount of a molecule of interest, preferably in "milligram" (abbreviated as "mg") produced by bacterial producer cells given as "gram" (abbreviated as "g") cell dry mass (abbreviated as "CDM"), e.g. [molecule of interest] mg/g CDM.

The "volumetric titre" is the amount of a molecule of interest, preferably in "milligram" (abbreviated as "mg") produced per 1 litre of culture medium given as "litre" (abbreviated as "L"), e.g. [molecule of interest] mg/L.

In accordance with the present invention, said helper protein is usually secreted by bacterial helper cells into the extracellular medium. "Extracellular medium" may - dependent on the context - be interchangeably used herein with the term "(bacterial) culture medium" or "(bacterial) cell culture". Namely, bacterial helper cells as described herein are constructed such that they secrete a helper protein due to equipping a helper protein with a bacterial secretion sequence. Accordingly, bacterial helper cells are capable of secreting a helper protein into extracellular medium. When bacterial helper cells as described herein are fermented, they secrete a helper protein into culture medium.

As has been found in context with the present invention, the addition of such helper cells can solve a number of problems faced when culturing bacterial producer cells for the production of a molecule of interest. Through expression of a helper protein that is secreted into the extracellular medium, the bacterial helper cells may be able to actively modify the composition of the extracellular medium in a manner responsive to fermentation-induced changes in the medium.

Secretion of a helper protein is achieved by a bacterial secretion sequence providing for the secretion of said helper protein into the extracellular medium.

By making use of the co-culture principle of the present invention, the efficiency of expression systems may be improved, for example, by breaking down unwanted by-products, thereby improving the purity of the reaction mixture and altering the final composition of the mixture to make the molecule of interest more readily available for further processing and/or extraction.

Further examples of problems that may be resolved by addition of helper cells may include provision of additional metabolic pathways to supply (precursor) molecules that the producer cell may lack (and need), detoxification of the extracellular medium through the breakdown of toxic by-products of the fermentation process, and substrate degradation to reduce extracellular waste that may be accumulating. By genetically modifying the helper cell to allow for the expression of helper proteins such as, e.g., exogenous enzymes, the range of problems that can be solved by the addition of a helper cell is greatly expanded.

The method and uses described and provided herein allows inter alia for the improved yield and/or productivity and therefore improved S-T-Y (space-time-yield) of the molecule(s) of interest. Further, the method and use described and provided herein facilitate downstream processes, since a helper protein needed, e.g. for processing a molecule of interest is already present and in the culture medium when bacterial producer cells are, e.g. lysed to harvest the molecule of interest. This is just an example and further examples for the facilitation of downstream processes are described herein.

In fact, in contrast to the principle of co-expressing of a molecule of interest and a helper protein as is done in the prior art, e.g. Balasundaram (2009), cited herein, the principle of co-culturing bacterial producer cells producing a molecule of interest and bacterial helper cells producing a helper protein results in higher specific and/or volumetric titres; see Figures 1a to d in comparison to Figure 4b or Figures 1 a to d in comparison to Figure 5b.

Moreover, when comparing the results shown in Figures 1a to d, Figures 2a to d and Figures 3 and b to the results shown in Figure 6, it is it is apparent that co-culture of bacterial producer and helper cells as described herein is advantageous in the production of a molecule of interest, e.g. a protein causing cell lysis, e.g. an endopeptidase, PETase or an antibody, in bacterial producer cells when said molecule of interest is produced by said bacterial producer cells, since bacterial helper cells producing a helper protein which aids in the production of said molecule of interest, e.g. a nuclease, which is secreted into the extracellular medium - here culture medium - degrades nucleic acids, e.g. dsDNA released from lysed bacterial producer cells. The advantage resides in the fact that otherwise released nucleic acids increase viscosity of the culture medium due to free nucleic acids, particularly dsDNA released from said lysed bacterial producer cells such that basically no downstream processing, e.g. harvesting bacterial producer cells or harvesting and/or purifying the molecule of interest is possible. Issues with viscosity arise usually when dsDNA is present in culture medium in an amount of about 400 µg/ml or higher. Issues with viscosity were observed in the prior art in the downstream processing of antibodies produced in bacterial cells and, accordingly, a nuclease was co-expressed in such bacterial cells. However, as is demonstrated herein, such co-expression results in low titres, while co-culturing of bacterial producer and helper cells achieves much higher titres. Accordingly, the present invention tackles issue with downstream processed two-fold, i.e., a helper protein is already present in the culture medium due to production and secretion by co-cultured bacterial helper cells and co-culture provides for high titres in comparison to co-expression.

Also, even though a molecule of interest which is produced by bacterial producer cells may not necessarily cause cell lysis which may require the presence of a helper protein produced any secreted by bacterial helper cells as described herein, e.g. a nuclease which reduces viscosity of the culture medium by degrading nucleic acids release from lysed bacterial producer cells, the concept of co-culture of bacterial producer cells and bacterial helper cells is anyway advantageous for downstream processes following the production phase.

Indeed, the prior art uses the concept of co-expression of e.g. an antibody fragment and a nuclease so as to have the nuclease available when harvesting the antibody fragment, because nucleic acids released from bacterial producer cells increase viscosity of culture medium and/or supernatant and thereby render any further process step potentially difficult, if not impossible; see Balasundaram (2009), cited herein. Yet, rather than co-expressing a molecule of interest and a helper protein in one and the same bacterial cell, the present invention applies the concept of co-culturing bacterial producer cells producing the molecule of interest and bacterial helper cells producing a helper protein. It is shown that the concept of co-culture provides for higher specific and/or volumetric titres; see Figures 1a to d in comparison to Figure 4b or Figures 1 a to d in comparison to Figure 5b

Accordingly, the concept of co-culturing of the present invention is not only advantageous for the production of a molecule of interest, e.g. a protein which may cause cell lysis when expressed by bacterial producer cells, because co-cultured bacterial helper cells provide for, e.g. a nuclease degrading nucleic acids released by lysed co-cultured bacterial producer cells. Rather, said concept of the present invention is also advantageous for the production of a molecule of interest which is subject to downstream processing including processing of said molecule of interest, e.g. cutting of a signal sequence or activating a protein by trimming the precursor form to become the mature form, e.g. in case of a pre-cursor form of a protease and a mature protease, because the bacterial helper cells provide during co-cultivation with the bacterial producer cells a helper protein which may be required for the production of the molecule of interest which may otherwise have to be added exogenously. Such exogenous addition may be more expensive

However, rather than adding a helper protein exogenously for, e.g. downstream processing of a molecule of interest, the present invention provides for an "in line" helper protein due to co-culturing bacterial producer cells and bacterial helper cells such that the helper protein produced by sad bacterial helper cells aids in the production of the molecule of interest produced by said bacterial producer cells. It is believed that it is both more effective and cheaper to produce such a helper protein "in line" (by bacterial helper cells) rather than adding it, perhaps in large amounts, exogenously. Hence, this is a further advantage of the concept of co-culture of bacterial producer and helper cells as described in the context of the present invention.

For example, bacterial producer cells which are co-cultured with bacterial helper cells as described herein may produce an pre-protein which needs to be processed in downstream processes by a protease produced by bacterial helper cells and which is secreted into the extracellular medium. Hence, as mentioned, presence of such a protease in the culture medium provides for processing the pre-protein into a mature protein. Due to co-culturing bacterial producer and helper cells, such processing may take place during fermentation and/or in subsequent downstream processes. However, in any event, downstream processing of the pre-protein can readily be done, since the protease is already present in the culture medium.

Moreover, it is believed that co-culture of bacterial producer cells producing such a pre-protein and bacterial helper cells producing such a protease results in higher specific and/or volumetric titres in comparison to co-expression of a pre-protein and a protease which is the usual approach in the prior art. Evidence comes from results shown in Figures 1a to d and Figures 2a to d in comparison to Figure 4b and Figure 5b, respectively, showing that co-culture is superior to co-expression.

For example, bacterial producer cells which are co-cultured with bacterial helper cells as described herein may produce an antibody which needs to be processed in downstream processes by binding to protein A, G or L produced by bacterial helper cells and which is secreted into the extracellular medium. Hence, as mentioned, presence of protein A, G or L in the culture medium binds to the antibody (or the antibody binds to protein A, G or L) which may facilitate, enriching and/or purifying such an antibody.

Due to co-culturing bacterial producer and helper cells, such enriching and/or purifying the antibody may take place during fermentation and/or in subsequent downstream processes. However, in any event, downstream processing of the antibody can readily be done, since protein A, G or L is already present in the culture medium.

Moreover, it is believed that co-culture of bacterial producer cells producing an antibody and bacterial helper cells producing protein A, G or L results in higher specific and/or volumetric titres in comparison to co-expression of an antibody and protein A, G or L which is the usual approach in the prior art. Evidence comes from results shown in Figures 1a to d and Figures 2a to d in comparison to Figure 4b and Figure 5b, respectively, showing that co-culture is superior to co-expression.

Of course, as also described herein, the helper protein produced by bacterial helper cells when bacterial producer cells produce an antibody, may alternatively or preferably additionally be a nuclease which degrades nucleic acids released from lysed bacterial producer cells (producing an antibody) during and/or after fermentation. Thus, in such a preferred embodiment, there is co-culture of bacterial producer cells producing an antibody and bacterial helper cells producing protein A, G or L and bacterial helper cells producing a nuclease.

As has further been found in context with the present invention, and as also further described herein, the production of molecules by co-culturing bacterial producer cells and bacterial helper cells may be further improved by decoupling growth and production of said bacterial producer cells and/or bacterial helper cells; compare Figures 1a to c with Figure 1d.

In one embodiment of the present invention, growth and production of both, bacterial producer cells and bacterial helper cells are decoupled. In another embodiment of the present invention, only growth and production of the bacterial producer cells is decoupled. In yet another embodiment of the present invention, only growth and production of the bacterial helper cells is decoupled. Decoupling growth and production is generally known in the art (see, e.g., WO 2016/174195, WO 2021/123402, WO 2007/071959, WO 97/34996, Mukherjee (2004), Appl Env Microbiol 70(5): 2005-3012, Rowe (1999), Appl Env Microbiol 65(6); 2710-2715) and also further described herein particularly in context with co-culturing bacterial producer cells and bacterial helper cells.

The term "growth" of the bacterial producer and/or helper cell as used herein means an increase of cell number due to cell division.

As used herein, the term "genetically engineered" generally means (unless specified otherwise herein) that the respective cell encodes and is capable to (stably) express a polynucleotide that it does naturally not contain and/or express. For example, such polynucleotide has been introduced into the respective cell. Preferably, such polynucleotide represents an entire gene or ORF, encoding a complete or relevant fragment of a protein. Such polynucleotide may be brought into the cell via technical or biological means, e.g., via transduction, transformation, transfection, or any other means that is suitable to introduce a polynucleotide into a cell, allowing (preferably stable) expression of that polynucleotide in said cell. Such methods are well known in the art and described, inter alia, in Molecular Cloning - A Laboratory Manual, 2nd Ed.,by Sambrook, Fritsch and Maniatis (Cold Spring Harbor Laboratory Press, 1989); DNA Cloning, Volumes I and II (D.N. Glover ed., 1985); Oligonucleotide Synthesis (M.J. Gait ed., 1984); Mullis et al., U.S. Patent No. 4,683,195; and Nucleic Acid Hybridization (B.D. Hames & S.J. Higgins eds., 1984). In accordance with the present invention, such polynucleotide may be introduced into the cell alone, i.e. solely the desired polynucleotide, or together with other polynucleotides (e.g., other genes, ORFs, promoter-, regulator- or enhancer-sequences, or other functional or non-functional nucleotides), either within a single molecule (e.g., a plasmid, cosmid, vector, or other forms) or on separate molecules. The respective cell which is "genetically engineered" in accordance with this invention may contain the introduced polynucleotide either on an extrachromosomal molecule (e.g., on a plasmid) or the polynucleotide may be (stably) integrated into the or a cell chromosome. The introduced polynucleotide may stand under the control of its own promoter or regulator or the like (introduced together with the polynucleotide), or under the control of a promoter or regulator or the like of the host cell (either alone or und common control together with an endogenous gene of the host cell).

In one aspect of the present invention, the molecule of interest produced by said bacterial producer cells may either harm the bacterial producer cell in a way that the bacterial producer cell reduces production of said molecule of interest over time, or have a negative impact on harvesting of the molecule of interest (e.g., by impacting viscosity of the medium). In such cases, in accordance with the present invention, it is preferred that the bacterial helper cells produce a helper protein that encounters such negative impacts, e.g., by supporting the bacterial producer cells to keep up the production of the molecules of interest, or by degrading substances that negatively impact harvesting the molecules of interest.

A "molecule of interest" as referred to herein is preferably a protein of interest, more preferably a protein of interest as described herein, e.g. an enzyme, an antibody, or a pre-protein.

It has been found by the present inventors that when producing a protein causing cell lysis in said bacterial producer cells when said molecule of interest is produced by said bacterial producer cells, it is advantageous to co-culture bacterial helper cells producing a helper protein which is secreted into the extracellular medium to aid in the production of said molecule of interest. Indeed, cell lysis and release of nucleic acids, in particular dsDNA from bacterial producer cells is an issue both for the fermentation and downstream processing due to the increase of viscosity.

"Cell lysis" when used herein is typically characterized by at least the release of nucleic acids, in particular dsDNA from bacterial producer cells due to leakiness and/or disruption of the cell membrane of the bacterial producer cell. Cell lysis includes unintentionally cell lysis due to the expression of a protein in a bacterial producer cell and also includes (intentional) breaking up of bacterial producer cells, e.g. to harvest a protein of interest.

The skilled person knows which proteins may cause (unintentional) cell lysis of bacterial producer cells. Such proteins may be a burden for bacterial producer cells, may interfere with protein synthesis, may interfere with metabolism, may interfere with DNA replication, protein processing, cell wall synthesis and the like, or may be misfolded.

As a specific example in accordance with the present invention an enzyme, e.g., a polytheylene terephthalate hydrolase (PETase, e.g., according to EC 3.1.1.101), e.g., the plastic-degrading polyester hydrolase PHL7, a member of the cutinase family of hydrolase enzymes, or a protease) may induce lysis of the bacterial producer cell membrane. It is also known from the prior art that an antibody produced by bacterial producer cells may cause cell lysis. The production of an antibody by bacterial producer cells as described herein is also envisaged. The loss of membrane integrity leads to release of DNA from the producing cell into the supernatant, resulting in an increase of viscosity of said supernatant. The high viscosity supernatant poses many challenges to subsequent processing - for example, the build-up of foam during post-production processing. As a result, taking accurate samples is challenging as the supernatant is too viscous to pipette, the biomass cannot be separated by centrifugation, and sterile filtration is time consuming and may still be insufficient to prevent the sample from blocking HPLC filters. A typical solution to this problem may be the application of an antifoaming agent, an agent which acts to reduce or hinder the formation of foam in a solution, but this poses additional issues as such an agent may act to inhibit the activity of the enzyme being produced by the fermentation process and add further labour and materials cost to the production process. In addition to posing challenges to processing, the accumulation of foam during bacterial fermentation is also known to reduce the efficiency of fermentation (Guo et al., MDPI Fermentation (2022), 8(2): 68). As has been found in context with the present invention, this can be overcome through the expression of a nuclease that is excreted into the medium from a bacterial helper cell. The presence of an extracellular nuclease will degrade the DNA present due to PHL-7 induced breakdown of bacterial producer cells, reducing the increase of supernatant viscosity that the accumulation of DNA would otherwise cause. Preventing the necessary use of the anti-foaming agent in turn reduces costs and prevents impairment of enzyme function. The principle of the use of an extracellular enzyme produced by a helper cell that degrades an extracellular impurity can be applied to a wide range of molecules due to the diversity of enzymes that may be expressed by virtue of the genetic engineering applied to the bacterial helper cells.

As has further been found in context with the present invention, there is a further benefit of decoupling growth from production of bacterial producer cells and/or bacterial helper cells to further enhance the method as described and provided herein. The decoupling of growth (also referred to herein as growth-decoupling) of a cell from the production of a molecule (e.g., protein or nucleotide) is a two stage process. According to the present invention, the growth, i.e. the propagation of the cells, may be (e.g., spatially and/or timely) separated from the production of the molecule (e.g., nucleotide or protein). In one bioreactor, the seed reactor, the cells may be kept under conditions that are ideal for their growth, i.e. propagation. No substantial production of the molecule (e.g., nucleotide or protein) is induced. Cells from the seed reactor may then be continuously transferred into a second bioreactor, the production reactor. The growth of the cell may then be inhibited and the production of the molecule (e.g., protein or nucleotide) be induced in the production reactor. It is also possible to apply a batch-process accordingly where cells are first cultivated in a growth-inducing environment and then transferred to a production-inducing environment. In context with the present invention it is also possible to apply a timely separation of growth induction and production induction, i.e. where first growth is induced in one reactor, then afterwards growth is inhibited and production induced in the same reactor. This concept is referred to herein as "growth-decoupling", the continuous process being preferred. Methods for decoupling growth and production particularly for continuous production are further described herein and also known in the art (see, e.g., WO 2021/123402). Using bacterial producer and/or helper cells as described herein in accordance with the present invention, a continuous production process in form a two stage production regime could by performed for extended periods of time.

The continuous fermentation process of the invention results in elevated continuous product yields and improved productivity. In this context, a fraction of the cells or the supernatant/culture medium of the production reaction may be continuously removed from the production reactor and the molecule (e.g., protein or nucleotide) of interest can be harvested. The fraction that is removed from the /production reactor may be preferably continuously replaced by fresh cells from the seed reactor. In accordance with the present invention, the principle of growth-decoupling may be further expanded to include the growth-decoupling of one or both, the bacterial producer cells and the bacterial helper cells, which may further enhance the rate of production and accumulation of the molecule of interest. In one embodiment of the present invention, growth and production of both, bacterial producer cells and bacterial helper cells are decoupled. In another embodiment of the present invention, only growth and production of the bacterial producer cells is decoupled. In yet another embodiment of the present invention, only growth and production of the bacterial helper cells is decoupled.

"Continuous fermentation", "continuous cultivation", or similar terms in context with growing bacterial cells in continuous processes as used herein may relate to operating conditions in which a liquid medium, such as a culture or nutrient medium, or a cells broth, is added to the fermenter, while culture fluid is released from the fermenter. The influx of the liquid medium is preferably constant at a certain rate or intermittently. The efflux of the culture fluid is preferably at the same rate so that the amount of liquid in the fermenter remains essentially constant. During continuous fermentation, biomass preferably remain essentially unchanged. In the steady state, the fermentation condition can be maintained, such as nutrient concentration, product concentration, constant pH, biomass according to the need. Reactors used for continuous fermentation can be stirred tank reactors or tubular reactors. A "continuous fermenter" or "reactor" as used herein is a fermenter or reactor that is suitable for continuous fermentation. (Initial) initiation of the continuous process of the invention may be done as following. Both, the seed and the production reactor, may be started as a batch culture followed by fed-batch until the necessary biomass concentration is achieved. Thus, during the initiation or, in other words, starting of the process of the invention, the microbial or bacterial host cells preferably are (both in the seed reactor and the production reactor) in an un-induced state with respect to the nucleotide sequence encoding a protein form a phage which inhibits growth of said bacterial cells. When the required cell density is reached, the production in the production reactor can be induced by addition of the inducer - in other words: The production reactor now is in an induced state with respect to the nucleotide sequence encoding a protein from, e.g., a phage which inhibits growth of said bacterial cells. Additionally, the continuous process can be started by transferring bacterial cells from the seed reactor to the production reactor, e.g. by starting a flow of culture medium into the seed reactor, thereby creating a flow from the seed reactor outlet to the production reactor inlet. Thus, in the continuous process of the invention, the cell growth is preferably inhibited in the production reactor. As described herein, the seed reactor is for propagation of the host cells. Further methods and examples to grow bacterial cells in continuous processes, also in context with decoupling growth and production of bacterial cells, are known in the art, see, e.g., WO 2021/1230402.

"Bioreactor" or "reactor", including the "seed bioreactor" or the "production bioreactor", as used herein refers to a reaction vessel for fermentation for production of cells and biosynthetic products, which may range in size from benchtop fermenters to industrial tanks. Bioreactors preferably allow automatic regulation of the flow of oxygen, culture medium and other nutrients, and maintaining the temperature and pH; they preferably minimize the potential for contamination and can produce a higher density of cells than can be produced in traditional cultures.

"Seed reactor" as used herein in the process and the system of the invention relates to the bioreactor that is used for propagation of the bacterial cell. In the seed reactor for the bacterial cell may be in an un-induced state with respect to the nucleotide sequence encoding a protein, e.g., from a phage, which inhibits growth of the bacterial cell. Otherwise, propagation in the seed reactor could not take place.

The term "un-induced state" with respect to the nucleotide sequence encoding a protein, e.g., from a phage which inhibits growth of the bacterial cell" relates to a microbial or bacterial cell, in which the promoter of the gene encoding the protein from a phage which inhibits growth of the bacterial cell has not been induced. As described herein, inhibition of the growth of the bacterial cell is inhibited by the expression of a protein, e.g., from a phage. Since the protein from a phage may be under the control of an inducible promoter, the un-induced state within the context of the invention thus means that the inducible promoter has not been induced. Un-induced in this context may mean, e.g. that in case of a chemically inducible promoter the inducer is not or not in sufficient concentration present in the culture medium, or that the effects of the inducer are impaired by other factors (e.g. the presence of glucose may suppress induction of a lac operon even in the inducer lactose is present) or in case of a temperaturesensitive promoter, the temperature for activation is not reached. This is preferred in the seed reactor. Accordingly, the bacterial cell preferably is cultured in an un-induced state with respect to the nucleotide sequence encoding a protein, e.g., from a phage which inhibits growth of said bacterial cell in the seed reactor for biomass production. Accordingly, the seed reactor preferably does not comprise or does not essentially comprise an inducer for the first inducible promoter. The first inducible promoter controls a nucleotide sequence encoding a protein, e.g., from a phage which inhibits growth of said bacterial cell. The seed reactor preferably is substantially free of an inducer for the first promoter, i.e. preferably in a concentration that does not induce a detectable expression of the protein, e.g., from a phage, which inhibits growth of said bacterial cell ("substantially free").

"Production reactor" as used herein in the process and the system of the invention relates to the bioreactor that is used for the production of the nucleotide or protein of interest. In the production reactor, the growth of the bacterial cells is inhibited by inducing the first promoter, and the production of the production of the nucleotide or protein of interest, which is under control of the second inducible promoter, is induced. Accordingly, the bacterial cell is in an induced state with respect to the nucleotide sequence encoding a protein, e.g., from a phage, which inhibits growth of said bacterial cell. Otherwise, production in the production reactor could not take place. Accordingly, the bacterial cell preferably is in an "induced state with respect to the nucleotide sequence encoding a protein from a phage" in the production reactor. Thus, in this case the inducible promoter is induced and the growth of the bacterial cell is inhibited. Accordingly, the bacterial cell is cultured in an induced state with respect to the nucleotide sequence encoding a protein, e.g., from a phage, which inhibits growth of said bacterial cell in the production reactor for production of said nucleotide sequence of interest by said bacterial cells.

When used herein, the term "growth of a bacterial (producer or host, respectively) cell" means that a bacterial (producer or host, respectively) cell is at least impaired in its growth compared to a bacterial (producer or host, respectively) cell which does not express protein (e.g., phage protein) that inhibits growth of a bacterial (producer or host, respectively) cell, and which comprises a nucleotide sequence encoding a RNA polymerase which is heterologous for said bacterial (producer or host, respectively) cell and, which comprises under the control of a promoter recognized by said RNA polymerase a nucleotide sequence which encodes a protein of interest. Notably, it is not excluded that such bacterial (producer or host, respectively) cell which does not express said growth inhibiting protein nevertheless comprises under the control of an inducible promoter a nucleotide sequence encoding a protein (e.g., from a phage) which inhibits growth of said bacterial (producer or host, respectively) cell. In fact, such a bacterial (producer or host, respectively) cell is a preferred reference cell, e.g. for determining the impairment of growth as described before. Put differently, when impairment of growth should be determined, the skilled artisan can easily compare a bacterial (producer or host, respectively) cell of the present invention when expression of said growth inhibiting protein is induced versus such a bacterial (producer or host, respectively) cell when expression of said growth inhibiting protein is not induced. When "growth of a bacterial (producer or host, respectively) cell" is inhibited as described herein, growth includes preferably transcription, DNA-replication and/or cell division. Accordingly, it is preferred that a growth inhibiting protein inhibits transcription, DNA-replication and/or cell division.

In context with the present invention, the method described and provided herein may further comprise providing a preparation of said bacterial producer cells and/or said bacterial helper cells. A "preparation" of a bacterial producer cell as used herein may be any preparation which is advantageously free of intact, living bacterial producer cells, but which has the capability of transcribing and translating a nucleotide sequence encoding a protein of interest, whereby said nucleotide sequence is under the control of a promoter recognized by the RNA polymerase which is heterologous for said bacterial producer cell from which the preparation is derived from. Such a preparation can, e.g., be prepared by mild lysis of bacterial producer cells or by mechanical forces such as subjecting such bacterial producer cells to a French press. Likewise, a "preparation" of a bacterial helper cell as used herein may be any preparation which is advantageously free of intact, living bacterial helper cells, but which has the capability of transcribing and translating a nucleotide sequence encoding a protein, whereby said nucleotide sequence is under the control of a promoter recognized by the RNA polymerase which is heterologous for said bacterial helper cell from which the preparation is derived from. Such a preparation can, e.g., be prepared by mild lysis of bacterial helper cells or by mechanical forces such as subjecting such bacterial helper cells to a French press.

As described, the concept of the present invention of co-culturing bacterial producer cells and bacterial helper cells is particularly useful for supporting the producer cells to produce more molecule(s) of interest, preferably in combination with decoupling growth and production of producer and/or helper cells as described herein. These cells may be genetically engineered to produce a molecule of interest (e.g., a protein or part thereof, for example an enzyme or an antibody or parts thereof) and/or a helper protein, respectively, wherein the helper protein aids the production of the molecule of interest. For example in accordance with the present invention, such a molecule of interest may be a protein, e.g., an enzyme. Specific examples of such enzymes include hydrolases such as, inter alia, polyethylene terephthalate hydrolase (PETase, e.g. according to EC 1.1.101), plastic-degrading polyester hydrolases such as PHL7, a member of the cutinase family of hydrolase enzymes, proteases, lipases, polyesterases and esterases.

Furthermore, in accordance with the invention, the helper protein expressed by said bacterial helper cells may reduce an impurity caused by said bacterial producer cells. For example, said impurity may be a nucleic acid, e.g., a DNA or RNA molecule or fragment thereof. Generally, as used herein, the term "nucleic acid" comprises DNA molecules, RNA molecules, oligonucleotide thiophosphates, substituted ribo-oligonucleotides or PNA molecules. Furthermore, the term "nucleic acid" may refer to DNA or RNA or hybrids thereof or any modification thereof that is known in the art (see, e.g., US 5525711, US 471 1955, US 5792608 or EP 302175 for examples of modifications). The molecules may be single- or doublestranded, linear or circular, natural or synthetic, and without any size limitation. For instance, the molecule may be genomic DNA, cDNA, mitochondrial DNA, mRNA, antisense RNA, ribozymal RNA or a DNA encoding such RNAs or chimeroplasts (Gamper, Nucleic Acids Research, 2000, 28, 4332 - 4339). In one specific example of the invention, said DNA is bacterial chromosomal DNA, mitochondrial DNA, or extrachromosomal DNA.

Furthermore in accordance with the invention, particularly where said helper protein expressed by said bacterial helper cells reduces an impurity caused by said bacterial producer cells, said helper protein may be a nuclease. In a more specific embodiment of the invention, said nuclease may be nuclease A from *Serratia marcescens,* Deoxyribonuclease I (DNase I), Nuclease P1, Mung Bean Nuclease, Lambda Exonuclease, Exonuclease III, T7 Endonuclease I, S1 Nuclease. More preferable is nuclease A from Serratia marcescens and variants thereof. A preferred amino acid sequence of nuclease A from Serratia marcescens is accessible at UniProt, accession number P13717.

Also, in accordance with the invention, the helper protein expressed by said bacterial helper cells may reduce an impurity caused by said bacterial producer cells. For example, said impurity may be lipopolysaccharide (LPS).

In accordance with the invention, particularly where said helper protein expressed by said bacterial helper cells reduces an impurity caused by said bacterial producer cells, wherein said impurity is lipopolysaccharide, said helper protein is a lipopolysaccharide binding protein, preferably a phage tail spike protein (TSP). TSP is capable of binding lipopolysaccharide.

Preferably, the phage tail spike protein (TSP) is T4-like bacteriophage gp12, T4 phage long tail fiber protein gp37, bacteriophage Bp7 receptor-recognizing protein gp38, E. coli phage DT571/2 tail fiber protein LtfA, E. coli phage DT571/2 tail fiber protein B, E. coli phage P2 tail fiber assembly protein G, E. coli phage Mu tail fiber assembly protein U', E34 phage TSP, Salmonella phage P22 TSP.

The T4 long tail fiber protein gp37 is has preferably the amino acid sequence accessible as P03744 in UniProt.

The bacteriophage Bp7 receptor-recognizing protein gp38 has preferably the amino acid sequence accessible as G3MUK4 in UniProt.

The E. coli phage DT571/2 tail fiber protein A has preferably the amino acid sequence accessible as AJA41761.1 in GenBank.

The E. coli phage DT571/2 tail fiber protein B has preferably the amino acid sequence accessible as AJA41760.1 in GenBank.

The E. coli phage P2 tail fiber assembly protein G has preferably the amino acid sequence accessible as P26699 in UniProt.

The E. coli phage Mu tail fiber assembly protein U' has preferably the amino acid sequence accessible as P0DJY5 in UniProt.

The E34 phage tail spike protein (TSP) has preferably the amino acid sequence accessible via Gene ID: 7353089 from the NCBI protein database

The Salmonella phage P22 tail spike protein (TSP) has preferably the amino acid sequence accessible as P12528 in UniProt.

In another embodiment of the invention, particularly where said helper protein expressed by said bacterial helper cells reduces an impurity caused by said bacterial producer cells, wherein said impurity is lipopolysaccharide, said helper protein is a lipopolysaccharide degrading enzyme.

Preferably, said lipopolysaccharide degrading enzyme is Cobetia amphilecti alkaline phosphatase, calf intestinal alkaline phosphatase, macrophage acyloxyacyl hydrolase (AOAH), polysaccharide lyase, e.g. from the phage LKA1 tailspike, or the lipopolysaccharide export system protein LptA from E. coli.

The Cobetia amphilecti alkaline phosphatase has preferably the amino acid sequence accessible as KGA01942.1 in GenBank.

The calf intestinal alkaline phosphatase has preferably the amino acid sequence accessible as P19111 in UniProt.

The macrophage acyloxyacyl hydrolase (AOAH) has preferably the amino acid sequence accessible as P28039 in UniProt.

The lipopolysaccharide export system protein LptA from E. coli has preferably the amino acid sequence accessible as P0ADV1 in UniProt.

In another aspect of the present invention, the bacterial producer cells need certain substances to produce a molecule of interest in desired amounts. Such substances may be, e.g., nutrients, pre-cursors of the molecule of interest to be produced, antibiotic additives to keep off non-desired commensals, or additives that relief harvesting of the molecule of interest from the medium. In such cases, in accordance with the present invention, it is preferred that the bacterial helper cells produce (and preferably secrete to the medium) a helper protein which is a respective pre-cursor or additive. Generally, in context with the present invention, in order to support production of the molecule(s) of interest by the bacterial producer cell, it is in most cases preferred that the molecule (e.g. and also referred to herein as helper protein) produced by the bacterial helper cell is secreted into the medium where it can best act to support production of the bacterial producer cell, e.g., by supplying (precursor) molecules that the producer cell may lack (and need), detoxify the extracellular medium through the breakdown of toxic by-products of the fermentation process, or degrade substances to reduce extracellular waste that may be accumulating in the medium. Accordingly, in one embodiment of the invention, said molecule (e.g. and also referred to herein as helper protein) produced by the bacterial helper cell is secreted by said bacterial helper cells.

In accordance with the present invention, the molecule of interest produced by said bacterial producer cells may be any molecule that can be produced by a bacterial cell. For example, it may be a protein or a pre-protein. In one specific example in accordance with the present invention, it may be a pre-protein. This may particularly be beneficial in context with the present invention in cases where the bacterial helper cell produces (and secretes into the medium) processes such pre-protein so that a protein of interest eventually accumulates which can then be harvested from the medium. Accordingly, in a specific embodiment of the invention, where the molecule of interest produced by said bacterial producer cells is a pre-protein, the helper protein expressed by bacterial helper cells processes said pre-protein produced by said bacterial producer cells. In a further embodiment of the invention, particularly where the molecule of interest produced by said bacterial producer cells is a pre-protein and said helper protein expressed by said bacterial helper cells processes said pre-protein produced by said bacterial producer cells, said helper protein produced (and preferably secreted) by the bacterial helper cell may be a protease. In a more specific embodiment of the present invention, said protease produced (and preferably secreted) by the bacterial helper cell may be an endopeptidase and/or exopeptidase. Such embodiments would allow proper processing of pre-proteins to yield proteins of interest. In a more specific embodiment of the present invention, said protease produced (and preferably secreted) by the bacterial helper cell may be an aspartate protease, cysteine protease, glutamate protease, metalloprotease, serine protease, threonine protease or methionine aminopeptidase.

In another aspect of the present invention, the molecule of interest produced by said bacterial producer cells is not easy to be directly harvested in pure form. In such cases, it may be desired in accordance with the present invention that the helper protein produced (and preferably secreted into the medium) may be a molecule that (specifically) binds to said molecule of interest to support harvesting said molecule of interest in pure form. For example, in one embodiment of the invention, said molecule of interest produced by said bacterial producer cells may be an antibody or fragment thereof. In this context, in one embodiment of the invention, said helper protein expressed by said bacterial helper cells (specifically) binds said antibody or fragment thereof produced by said bacterial producer cells. In other words, as used herein, particularly where the molecule of interest is an antibody or fragment thereof, said helper protein may be a recognition molecule capable of (specifically) binding said antibody or fragment thereof. In one embodiment of the invention, particularly where said molecule of interest produced by said bacterial producer cells is an antibody and where helper protein expressed by said bacterial helper cells binds said antibody produced by said bacterial producer cells, said helper protein is protein A, G or L.

A "recognition molecule" as used herein may be a polypeptide which comprises one or more binding domains capable of binding to a target epitope. A recognition molecule, so to say, provides the scaffold for said one or more binding domains so that said binding domains can bind/interact with a given target structure/antigen/epitope. The term "binding domain" characterizes in connection with the present invention a domain of a polypeptide which specifically binds/interacts with a given target epitope. An "epitope" is antigenic and thus the term epitope is sometimes also referred to herein as "antigenic structure" or "antigenic determinant". Thus, the binding domain is an "antigen-interaction-site". The term "antigen-interaction-site" defines, in accordance with the present invention, a motif of a polypeptide, which is able to specifically interact with a specific antigen or a specific group of antigens, e.g. the identical antigen in different species. Said binding/interaction is also understood to define a "specific recognition". Specific binding as used herein is considered to be effected by specific motifs in the amino acid sequence of the antibody or fragment thereof produced by the bacterial producer cell and the recognition molecule produced by the bacterial helper cell bind to each other as a result of their primary, secondary or tertiary structure as well as the result of secondary modifications of said structure. The specific interaction of the interaction-site of the antibody or fragment thereof with its specific recognition molecule may result as well in a simple binding of said site to the recognition molecule. Moreover, the specific interaction of the interaction-site with its specific recognition molecule may alternatively result in the initiation of a signal, e.g. due to the induction of a change of the conformation of the recognition molecule, an oligomerization of the antigen, etc. Typically, binding is considered "specific" when the binding affinity is higher than 10-6M. Preferably, binding is considered specific when binding affinity is about 10-11 to 10-8 M (KD), preferably of about 10-11 to 10-9 M. If necessary, nonspecific binding can be reduced without substantially affecting specific binding by varying the binding conditions. Whether the recognition molecule specifically reacts as defined herein above can easily be tested, inter alia, by comparing the reaction of said recognition molecule with an epitope with the reaction of said recognition molecule with (an) other protein(s). An "antibody" as used herein is a protein comprising one or more polypeptides (comprising one or more binding domains, preferably antigen binding domains) substantially or partially encoded by immunoglobulin genes or fragments of immunoglobulin genes. The term "immunoglobulin" (Ig) is used interchangeably with "antibody" herein. The recognized immunoglobulin genes include the kappa, lambda, alpha, gamma, delta, epsilon and mu constant region genes, as well as myriad immunoglobulin variable region genes. In particular, an "antibody" when used herein, is typically tetrameric glycosylated proteins composed of two light (L) chains of approximately 25 kDa each and two heavy (H) chains of approximately 50 kDa each. Two types of light chain, termed lambda and kappa, may be found in antibodies. Depending on the amino acid sequence of the constant domain of heavy chains, immunoglobulins can be assigned to five major classes: A, D, **E,** G, and M, and several of these may be further divided into subclasses (isotypes), e.g., IgG1, IgG2, IgG3, IgG4, IgA1, and IgA2. An IgM antibody consists of 5 of the basic heterotetramer unit along with an additional polypeptide called a J chain, and contains 10 antigen binding sites, while IgA antibodies comprise from 2-5 of the basic 4-chain units which can polymerize to form polyvalent assemblages in combination with the J chain. In the case of IgGs, the 4-chain unit is generally about 150,000 daltons. Each light chain includes an N-terminal variable (V) domain (VL) and a constant (C) domain (CL). Each heavy chain includes an N-terminal V domain (VH), three or four C domains (CHs), and a hinge region. The constant domains are usually not involved directly in binding an antibody to a recognition molecule. As used herein, the term "antibody" does not only refer to an immunoglobulin (or intact antibody), but also to a fragment thereof, and encompasses any polypeptide comprising an antigen-binding fragment or an antigen-binding domain. Preferably, the fragment such as Fab, F(ab')2, Fv, scFv, Fd, dAb, and other antibody fragments that retain antigen-binding function. Typically, such fragments would comprise an antigen-binding domain and have the same properties as the antibodies described herein. The term "antibody" as used herein includes antibodies that compete for binding to the same epitope as the epitope bound by the antibodies of the present invention, preferably obtainable by the methods for the generation of an antibody as described herein elsewhere. In accordance with the present invention, the term "antibody" also includes but is not limited to polyclonal, monoclonal, monospecific, polyspecific such as bispecific, nonspecific, humanized, human, single-chain, chimeric, synthetic, recombinant, hybrid, mutated, grafted, and in vitro generated antibodies, with a polyclonal antibody being preferred. Said term also includes domain antibodies (dAbs) and nanobodies. Furthermore, the term "antibody" as employed in the invention also relates to derivatives or variants of the antibodies described herein which display the same specificity as the described antibodies. Examples of "antibody variants" include humanized variants of non-human antibodies, "affinity matured" antibodies (see, e.g., Hawkins et al., J Mol Biol (1992), 254, 889-896; and Lowman et al., Biochemistry (1991), 30: 10832- 10837) and antibody mutants with altered effector function (s) (see, e.g., US Patent 5, 648, 260). The term "antibody" further includes antibody-mimetics, such as single domain antibodies (VHHs), DARPins or affibodies, anticalins, avimers, affilins etc.

As described and provided in accordance with the present invention, there is a further benefit of decoupling growth from production of bacterial producer cells and/or bacterial helper cells to further enhance the method as described and provided herein. The decoupling of growth (also referred to herein as growth-decoupling) of a cell from the production of a molecule (e.g., protein or nucleotide) is a two stage process. According to the present invention, the growth, i.e. the propagation of the cells, may be (e.g., spatially and/or timely) separated from the production of the molecule (e.g., nucleotide or protein). Decoupling growth and production is generally known in the art (see, e.g., WO 2016/174195 or WO 2021/123402) and also further described herein, also in context with co-culturing bacterial producer cells and bacterial helper cells. In this context, it may be desired in accordance with the present invention that the bacterial producer cells and/or the bacterial helper cells are equipped such that growth and production can be decoupled. General methods are known and can be found, e.g., in (WO 2016/174195 and WO 2021/123402, as well as Mukherjee et al., cited herein; Rowe et al., cited herein; WO 97/34996 or WO 2007/071959 or Clavier (2024), Biotech Bioengineering, doi: 10.1002/bit.28756.). For example, in one embodiment of the present invention, said bacterial producer cells and/or bacterial helper cells may be genetically engineered with a construct which inhibits growth of said bacterial cells. This allows switching OFF the host cell's proliferation in the production reactor and allows high yield production in the production reactor. Generally, in context with the present invention, growth of bacterial producer and/or helper cells can be inhibited by inhibiting RNA polymerase in cells (e.g., *E. coli*)*.* In turn, production in bacterial producer and/or helper cells can be increased by inhibiting RNA polymerase in cells. For example, in order to genetically engineer a bacterial producer cell to inhibit growth accordingly, the bacterial producer cell may be transformed with a nucleotide sequence encoding a protein from a phage (for example, a protein that inhibits the RNA polymerase of the bacterial cell, e.g., gp0.7 and/or Gp2 from phage T7) which inhibits growth of said bacterial producer cell as defined herein, and optionally also with a nucleotide sequence encoding phage (e.g., T7) RNA polymerase, and a nucleotide sequence encoding a molecule (e.g., protein) of interest. Likewise, in order to genetically engineer a bacterial helper cell to inhibit growth accordingly, the bacterial helper cell may be transformed with a nucleotide sequence encoding a protein from a phage which inhibits growth of said bacterial helper cell as defined herein with a nucleotide sequence encoding a phage protein, e.g., T7 RNA polymerase, and a nucleotide sequence encoding a helper molecule (e.g., protein). For example, bacteriophage T7 uses its proteins gp0.7 and Gp2 to shut off *E.coli* RNA polymerase after infection. Immediately after infection early viral class I genes of bacteriophage T7, under control of bacterial promoters, are expressed, such as T7 RNA polymerase which is highly specific for viral genes under control of the T7 promoter. Among the class I genes is Gp0.7, which phosphorylates inter alia *E*. *coli* RNA polymerase resulting in transcription termination of early genes and in switching from host to viral RNA polymerase. Subsequently, the viral gene Gp2 is expressed, binding to and further inhibiting the beta subunit of the host RNA polymerase. Together Gp0.7 and/or Gp2 inhibit *E.coli* RNA polymerase and thereby cellular proliferation. Accordingly, in accordance with the present invention, proteins gp0.7 and/or Gp2 from phage T7 may be used to inhibit growth of bacterial producer and/or helper cells. Accordingly, in accordance with the present invention, bacterial producer and/or helper cells may be genetically engineered (transformed) with nucleic acids encoding gp0.7 and/or Gp2 from phage T7. Further suitable phage proteins to be used in accordance with the present invention to inhibit growth of bacterial cells include, for example, Nun, Gp6, Gp8 or A*, Bacillus phage SPO1 GP40 SPO1 GP40, Staphylococcus phage G1 GP67, Thermus thermophilus phage P23-45 GP39, Enterobacteria phage PhiEco32 GP79, Xanthomonas oryzae bacteriophage Xp10 P7 protein, Enterobacteria phage T4 Ale protein, Enterobacteria phage T4Asia or Bacillus subtilis ykzG protein. The term "transform" as used herein means alteration of the genotype of a cell by introducing a nucleotide sequence. The nucleotide sequence does not necessarily originate from a different source, but it will, at some point, have been external to the cell into which it is to be introduced.

In accordance with the present invention, each of said bacterial producer cells and said bacterial helper cells may be cultivated jointly or first in a separate pre-culture, preferably in a separate pre-culture. That is, both, bacterial producer cells and bacterial helper cells may thus be cultivated first to induce growth, either jointly or (preferably) in separate pre-cultures. In accordance with the present invention, after separate pre-culturing, said bacterial producer cells and said bacterial helper cells may subsequently be mixed in a pre-defined ratio for being co-cultured. Suitable methods to culture cells depend on the type of cells used and are generally known in the art.

Pre-defined ratios are preferably made by making use of OD600 (nm) of the bacterial producer and helper cells. Accordingly, the skilled person determines the OD600 of both bacterial producer and bacterial helper cells and mixes them in pre-defined ratios. Pre-defined ratios may be determined by the following test experiment: the skilled person mixes bacterial producer and helper cells in various ratios, e.g. 1 OD600 of bacterial producer cells and 0.1, 0.2, 0.3, 0.4, 0.5, 0.6, 0.7, 0.8, 0.9, 1.0%, etc. OD600 of bacterial helper cells, whereby the 0.1% OD600 of bacterial helper cells is in relation to the OD600 of bacterial producer cells; co-cultures such mixtures over a certain period of time, and determines by way of, e.g. determining the titre of the molecule of interest or culture medium characteristics, e.g. viscosity, the best titre and/or culture medium characteristics, dependent on the needs. Accordingly, on the basis of the outcome of the test experiment, the skilled person will be able to determine the pre-defined ratio of bacterial producer and bacterial helper cells.

Accordingly, suitable ratios may be 1 OD600 of bacterial producer cells and 0.1% OD600 of bacterial helper cells; 1 OD600 of bacterial producer cells and 0.2% OD600 of bacterial helper cells; 1 OD600 of bacterial producer cells and 0.3% OD600 of bacterial helper cells; 1 OD600 of bacterial producer cells and 0.4% OD600 of bacterial helper cells; 1 OD600 of bacterial producer cells and 0.5% OD600 of bacterial helper cells; 1 OD600 of bacterial producer cells and 0.6% OD600 of bacterial helper cells; 1 OD600 of bacterial producer cells and 0.7% OD600 of bacterial helper cells; 1 OD600 of bacterial producer cells and 0.8% OD600 of bacterial helper cells; 1 OD600 of bacterial producer cells and 0.9% OD600 of bacterial helper cells; 1 OD600 of bacterial producer cells and 1% OD600 of bacterial helper cells; 1 OD600 of bacterial producer cells and 2, 5, 6, 7, 8, 9, 10, 15, 20, 25, 30, 35, 40, 45, 50, 55, 60, 65, 70, 75, 80, 85, 90, 95 or 100% OD600 of bacterial helper cells.

It goes without saying that, e.g. 1 OD600 of bacterial producer cells and 0.1% OD600 of bacterial helper cells corresponds to 10 OD600 of bacterial producer cells and 1% OD600 bacterial helper cells, etc.

Generally, in context with the present invention, a bacterial producer or helper cell may be any bacterial cell that is able to produce a molecule of interest. Preferably, it is a prokaryotic cell. Preferred examples of a prokaryotic bacterial producer (or helper) cell in accordance with the present invention include *E. coli,* Pseudomonas species, Salmonella species, Bacillus species, Lactobacillus species, Corynebacterium species, Microbacterium species, Actinomycetes species or Vibrio species, preferably *E. coli.*

A large number of suitable methods exist in the art to produce molecules of interest (e.g., proteins) in bacterial producer cells of the invention. Conveniently, the produced protein is harvested from the culture medium, lysates of the cultured host cell or from isolated (biological) membranes by established techniques. For example, an expression cassette comprising, inter alia, the nucleotide sequence encoding the protein of interest can be synthesized by PCR and inserted into the expression vector. Subsequently, a cell may be transformed with the expression vector. Thereafter, the cell is cultured to produce/express the desired protein(s), which is/are isolated and purified. For example, the product may be recovered from the host cell and/or culture medium by conventional procedures including, but not limited to, cell lysis, breaking up host cells, centrifugation, filtration, ultra-filtration, extraction, evaporation, spray drying or precipitation. Purification may be performed by a variety of procedures known in the art including, but not limited to, chromatography (e.g. ion exchange, affinity, hydrophobic, chromatofocus, and size exclusion), electrophoretic procedures (e.g., preparative isoelectric focusing), differential solubility (e.g. ammonium sulfate precipitation) or extraction.

The invention further relates to the use of co-cultured bacterial producer cells and bacterial helper cells as described herein, wherein said bacterial producer cells and/or bacterial helper cells are growth and production decoupled (preferably of both), wherein said bacterial producer cells are genetically engineered to produce said molecule of interest as described herein, and said bacterial helper cells are genetically engineered to produce a (helper) protein as described herein aiding in the production of said molecule of interest, for the production of a molecule of interest.

The invention further relates to a bacterial consortium comprising bacterial producer cells which are genetically engineered as described herein to produce a molecule of interest as described herein and bacterial helper cells which are genetically engineered to produce a (helper) protein as described herein aiding in the production of said molecule of interest, wherein each of said bacterial producer cells and bacterial helper cells as described herein is equipped such that growth and production can be decoupled as described herein. In one embodiment of the present invention, said bacterial consortium, each of said bacterial producer cells and bacterial helper cells is genetically engineered with a construct which inhibits growth of said bacterial cells. This allows switching OFF the host cell's proliferation in the production reactor and allows high yield production in the production reactor. Generally, in context with the present invention, growth of bacterial producer and/or helper cells can be inhibited by inhibiting RNA polymerase in cells (e.g., *E. coli*)*.* In turn, production in bacterial producer and/or helper cells can be increased by inhibiting RNA polymerase in cells. For example, in order to genetically engineer a bacterial producer cell to inhibit growth accordingly, the bacterial producer cell may be transformed with a nucleotide sequence encoding a protein from a phage (for example, a protein that inhibits the RNA polymerase of the bacterial cell, e.g., gp0.7 and/or Gp2 from phage T7) which inhibits growth of said bacterial producer cell as defined herein, and optionally also with a nucleotide sequence encoding phage (e.g., T7) RNA polymerase, and a nucleotide sequence encoding a molecule (e.g., protein) of interest. Likewise, in order to genetically engineer a bacterial helper cell to inhibit growth accordingly, the bacterial helper cell may be transformed with a nucleotide sequence encoding a protein from a phage which inhibits growth of said bacterial helper cell as defined herein with a nucleotide sequence encoding a phage protein, e.g., T7 RNA polymerase, and a nucleotide sequence encoding a helper molecule (e.g., protein). For example, bacteriophage T7 uses its proteins gp0.7 and Gp2 to shut off *E.coli* RNA polymerase after infection. Immediately after infection early viral class I genes of bacteriophage T7, under control of bacterial promoters, are expressed, such as T7 RNA polymerase which is highly specific for viral genes under control of the T7 promoter. Among the class I genes is Gp0.7, which phosphorylates inter alia *E*. *coli* RNA polymerase resulting in transcription termination of early genes and in switching from host to viral RNA polymerase. Subsequently, the viral gene Gp2 is expressed, binding to and further inhibiting the beta subunit of the host RNA polymerase. Together Gp0.7 and/or Gp2 inhibit *E.coli* RNA polymerase and thereby cellular proliferation. Accordingly, in accordance with the present invention, proteins gp0.7 and/or Gp2 from phage T7 may be used to inhibit growth of bacterial producer and/or helper cells. Accordingly, in accordance with the present invention, bacterial producer and/or helper cells may be genetically engineered (transformed) with nucleic acids encoding gp0.7 and/or Gp2 from phage T7. Further suitable phage proteins to be used in accordance with the present invention to inhibit growth of bacterial cells include, for example, Nun, Gp6, Gp8 or A*, Bacillus phage SPO1 GP40 SPO1 GP40, Staphylococcus phage G1 GP67, Thermus thermophilus phage P23-45 GP39, Enterobacteria phage PhiEco32 GP79, Xanthomonas oryzae bacteriophage Xp10 P7 protein, Enterobacteria phage T4 Ale protein, Enterobacteria phage T4Asia or Bacillus subtilis ykzG protein. The term "transform" as used herein means alteration of the genotype of a cell by introducing a nucleotide sequence. The nucleotide sequence does not necessarily originate from a different source, but it will, at some point, have been external to the cell into which it is to be introduced.

In accordance with the present invention, said bacterial producer cells of said bacterial consortium may produce an enzyme and said bacterial helper cells of said bacterial consortium may produce a nuclease. Specific examples of such enzymes include hydrolases such as, inter alia, polyethylene terephthalate hydrolase (PETase, e.g. according to EC 1.1.101), plastic-degrading polyester hydrolases such as PHL7, a member of the cutinase family of hydrolase enzymes, or proteases or proteases, lipases, polyesterases and esterases. Specific examples of such nucleases may include nuclease A (benzonase) from *Serratia marcescens,* Deoxyribonuclease I (DNase I), Nuclease P1, Mung Bean Nuclease, Lambda Exonuclease, Exonuclease III, T7 Endonuclease I, S1 Nuclease. Processes to produce Nuclease A (benzonase) in suitable bacterial producer cells are known in the art and also described in, e.g., WO 2011/015327. In another embodiment of the present invention, said bacterial producer cells of said bacterial consortium produce a pre-protein as described herein and said bacterial helper cells of said bacterial consortium produce a protease. In this context, for example said protease produced (and preferably secreted) by the bacterial helper cell may be an endopeptidase and/or exopeptidase. This would also allow proper processing of pre-proteins to yield proteins of interest. In more specific embodiments of the present invention, said protease produced (and preferably secreted) by the bacterial helper cell may be an aspartate protease, cysteine protease, glutamate protease, metalloprotease, serine protease, threonine protease or methionine aminopeptidase.

In another embodiment of the present invention, said bacterial producer cells of said bacterial consortium produce an antibody as described herein and said bacterial helper cells of said bacterial consortium produce protein A, G or L.

The embodiments which characterize the present invention are described herein, shown in the Figures, illustrated in the Examples, and reflected in the claims.

It must be noted that as used herein, the singular forms "a", "an", and "the", include plural references unless the context clearly indicates otherwise. Thus, for example, reference to "a reagent" includes one or more of such different reagents and reference to "the method" includes reference to equivalent steps and methods known to those of ordinary skill in the art that could be modified or substituted for the methods described herein.

Unless otherwise indicated, the term "at least" preceding a series of elements is to be understood to refer to every element in the series. Those skilled in the art will recognize, or be able to ascertain using no more than routine experimentation, many equivalents to the specific embodiments of the invention described herein. Such equivalents are intended to be encompassed by the present invention.

The term "and/or" wherever used herein includes the meaning of "and", "or" and "all or any other combination of the elements connected by said term".

The term "about" or "approximately" as used herein means within 20%, preferably within 10%, and more preferably within 5% or 2% of a given value or range, and also comprise the respective exact numeric value.

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integer or step. When used herein the term "comprising" can be substituted with the term "containing" or "including" or sometimes when used herein with the term "having".

When used herein "consisting of" excludes any element, step, or ingredient not specified in the claim element. When used herein, "consisting essentially of" does not exclude materials or steps that do not materially affect the basic and novel characteristics of the claim.

In each instance herein any of the terms "comprising", "consisting essentially of" and "consisting of" may be replaced with either of the other two terms.

When used herein, the term "essentially", e.g., "essentially free of" or "essentially non-[...]" or the like, the term also includes the absolute condition, i.e. "free of", "non-[...]" or the like, respectively.

It should be understood that this invention is not limited to the particular methodology, protocols, and reagents, etc., described herein and as such can vary. The terminology used herein is for the purpose of describing particular embodiments only, and is not intended to limit the scope of the present invention, which is defined solely by the claims.

All publications and patents cited throughout the text of this specification (including all patents, patent applications, scientific publications, manufacturer's specifications, instructions, etc.), whether supra or infra, are hereby incorporated by reference in their entirety. Nothing herein is to be construed as an admission that the invention is not entitled to antedate such disclosure by virtue of prior invention. To the extent the material incorporated by reference contradicts or is inconsistent with this specification, the specification will supersede any such material.

The present invention can also be characterized by the following items:
1. A method for the production of a molecule of interest, comprising co-culturing bacterial producer cells and bacterial helper cells,
   wherein said bacterial producer cells are genetically engineered to produce said molecule of interest, and
   said bacterial helper cells are genetically engineered to produce a helper protein that is secreted into the extracellular medium,
   thereby aiding in the production of said molecule of interest.
2. The method of item 1, further comprising providing a preparation of said bacterial producer cells and/or said bacterial helper cells.
3. The method of item 1 or 2, wherein said method comprises decoupling growth and production of said bacterial producer cells and/or said bacterial helper cells.
4. The method of any one of items 1 to 3, wherein said molecule of interest produced by said bacterial producer cells is a protein causing cell lysis in said bacterial producer cells when said molecule of interest is produced by said bacterial producer cells.
5. The method of any one of items 1 to 4, wherein said molecule of interest produced by said bacterial producer cells is an enzyme or antibody.
6. The method of item 5, wherein said enzyme is a polyethylene terephthalate hydrolase (PETase) [EC 3.1.1.101] or a protease.
7. The method of any one of the preceding items, wherein said helper protein expressed by said bacterial helper cells reduces an impurity caused by said bacterial producer cells.
8. The method of item 7, wherein said impurity is a nucleic acid.
9. The method of item 8, wherein said nucleic acid is DNA or RNA.
10. The method of item 9, wherein said DNA is bacterial chromosomal DNA, mitochondrial DNA or extrachromosomal DNA.
11. The method of any one of items 7 to 10, wherein said helper protein is a nuclease.
12. The method of item 11, wherein said nuclease is nuclease A from Serratia marcescens.
13. The method of item 7, wherein said impurity is lipopolysaccharide.
14. The method of item 7 or 13, wherein said helper protein is a lipopolysaccharide binding protein, preferably a phage tail spike protein (TSP), preferably T4-like bacteriophage gp12, T4 phage long tail fiber protein gp37, bacteriophage Bp7 receptor-recognizing protein gp38, E. coli phage DT571/2 tail fiber protein LtfA, E. coli phage DT571/2 tail fiber protein B, E. coli phage P2 tail fiber assembly protein G, E. coli phage Mu tail fiber assembly protein U', E34 phage TSP, Salmonella phage P22 TSP.
15. The method of item 7 or 13, wherein said helper protein is a lipopolysaccharide degrading enzyme, preferably Cobetia amphilecti alkaline phosphatase, calf intestinal alkaline phosphatase, macrophage acyloxyacyl hydrolase (AOAH), polysaccharide lyase, e.g. from the phage LKA1 tailspike, or lipopolysaccharide export system protein LptA from E. coli.
16. The method of item 1, wherein said molecule of interest produced by said bacterial producer cells is a pre-protein.
17. The method of item 1 or 16, wherein said helper protein expressed by said bacterial helper cells processes said pre-protein produced by said bacterial producer cells.
18. The method of item 17, wherein said helper protein is a protease.
19. The method of item 18, wherein said protease is an endopeptidase and/or exopeptidase.
20. The method of item 18 or 19, wherein said protease is an aspartate protease, cysteine protease, glutamate protease, metalloprotease, serine protease, threonine protease or methionine aminopeptidase.
21. The method of item 1, wherein said molecule of interest produced by said bacterial producer cells is an antibody.
22. The method of item 1 or 21, wherein said helper protein expressed by said bacterial helper cells binds said antibody produced by said bacterial producer cells.
23. The method of item 22, wherein said helper protein is protein A, G or L.
24. The method of any one of the preceding items, wherein said bacterial producer cells and/or bacterial helper cells are equipped such that growth and production can be decoupled.
25. The method of any one of the preceding items, wherein said bacterial producer cells and/or bacterial helper cells are genetically engineered with a construct which inhibits growth of said bacterial cells.
26. The method of any one of the preceding items, wherein each of said bacterial producer cells and said bacterial helper cells is cultivated in a separate pre-culture.
27. The method of any one of the preceding items, wherein, after separate pre-culturing, said bacterial producer cells and said bacterial helper cells are mixed in a pre-defined ratio for being co-cultured.
28. Use of co-cultured bacterial producer cells and bacterial helper cells, wherein said bacterial producer cells and/or bacterial helper cells are growth and production decoupled, wherein said bacterial producer cells are genetically-engineered to produce said molecule of interest, and said bacterial helper cells are genetically-engineered to produce a helper protein aiding in the production of said molecule of interest, for the production of a molecule of interest.
29. A bacterial consortium comprising bacterial producer cells which are genetically-engineered to produce a molecule of interest and bacterial helper cells which are genetically-engineered to produce a protein aiding in the production of said molecule of interest, wherein each of said bacterial producer cells and bacterial helper cells is equipped such that growth and production can be decoupled.
30. The bacterial consortium of item 29, wherein each of said bacterial producer cells and bacterial helper cells is genetically engineered with a construct which inhibits growth of said bacterial cells.
31. The bacterial consortium of item 29 or 30, wherein said bacterial producer cells produce an enzyme and said bacterial helper cells produce a nuclease.
32. The bacterial consortium of item 29 or 30, wherein said bacterial producer cells produce a pre-protein and said bacterial helper cells produce a protease.
33. The bacterial consortium of item 29 or 30, wherein said bacterial producer cells produce an antibody and said bacterial helper cells produce protein A, G or L.

### VI. EXAMPLES

For the pre-culture, a 500 mL baffled bottom flask was filled with 30 mL of semi-synthetic medium (3 g/L CDM) containing 50 µg/mL kanamycin for each strain (Co-cultivation: producer and helper cells, Co-expression: producer cells). As an alternative to the semi-synthetic medium, LB medium may be used.

The pre-cultures were inoculated with 2 mL of working cell bank each and incubated for 8 h at 37 °C and 200 rpm.

The OD was measured and the amount of cell suspension equaling 25 OD units was used to inoculate 500 mL of batch medium (see Table below) in the DASGIP reactor system (Eppendorf SE, Hamburg, Germany).

For inoculation of co-cultivation processes producer cells and helper cells were mixed in the ratio of 99:1. However, any other ratio is possible as described herein.

The temperature was kept at 37 (+/- 0.2) °C, the pH control was set to 7 (+/- 0.1) and regulated by dosing 12.5% ammonia solution.

The dissolved oxygen (DO) cascade was designed to maintain the DO at 30% by first accelerating the stirrer speed (400 - 1200 rpm), then increasing the air flow rate (10 - 200 sL/h) and finally increasing the oxygen concentration in the gas flow (21 - 100%).

Foaming in the reactor was kept under control by dosing antifoam agent (1:10 diluted, Struktol J673A, Schill+Seilacher, Böblingen, Germany) when needed.

The batch phase ended after 8-10 h with a cell dry weight (CDW) of approximately 10 g/L.

Then the first feed phase (feed medium composition see Table 1, below), with an exponential feed profile (µ=0.135 h⁻¹), was initiated. At the end of the exponential feed phase 40-50 g/L of CDW were reached. Subsequently the feed was set to a linear feed profile with a fixed feed rate (resulting in a theoretical growth rate of µ=0.05 h⁻¹) and the temperature was set to 30 C.

With the start of the linear feed, the growth of the host cells was halted by addition of 10 mM arabinose to express the T7 phage protein Gp2 and in parallel the enzyme expression was induced with IPTG (0.4 µmol/g CDW in the initial fermentation process) thereby decoupling growth and recombinant protein production.

The first sample was taken right before induction as a reference and to determine possible basal expression of the molecule of interest. Further samples were drawn according to sampling plan represented by the graph (usually every 3h post induction).

**Table 1**

| Fermentation media | | | |
|---|---|---|---|
| Order of addition | Media component | Amount Batch | Amount Feed |
| 1 | KH2PO4 | 2.1 g/L | 8.3 g/L |
| 1 | 85% H3PO4 | 0.7 g/L | 2.8 g/L |
| 2 | Yeast extract | 1.5 g/L | --- |
| 3 | Na-citrate | 0.4 g/L | 4.1 g/L |
| 4 | MgCl2*6H2O | 0.5 g/L | 4.6 g/L |
| 5 | CaCl2*2H2O | 0.2 g/L | 2.0 g/L |
| 6 | Trace elements | 500 µl | 5 mL |
| 7 | (NH4)2SO4 | 5.0 g/L | --- |
| 8 | Glucose | 28.6 g/L | 285.7 g/L |

## Claims

1. A method for the production of a molecule of interest, comprising co-culturing bacterial producer cells and bacterial helper cells,
wherein said bacterial producer cells are genetically engineered to produce said molecule of interest, and
said bacterial helper cells are genetically engineered to produce a helper protein that is secreted into the extracellular medium,
thereby aiding in the production of said molecule of interest.

2. The method of claim 1, wherein said method comprises decoupling growth and production of said bacterial producer cells and/or said bacterial helper cells.

3. The method of any one of claims 1 to 2, wherein said molecule of interest produced by said bacterial producer cells is a protein causing cell lysis in said bacterial producer cells when said molecule of interest is produced by said bacterial producer cells.

4. The method of any one of claims 1 to 3, wherein said molecule of interest produced by said bacterial producer cells is an enzyme or antibody.

5. The method of any one of the preceding claims, wherein said helper protein expressed by said bacterial helper cells reduces an impurity caused by said bacterial producer cells.

6. The method of claim 5, wherein said helper protein is a nuclease or a lipopolysaccharide binding protein or a lipopolysaccharide degrading enzyme.

7. The method of claim 1, wherein said molecule of interest produced by said bacterial producer cells is a pre-protein.

8. The method of claim 1 or 7, wherein said helper protein expressed by said bacterial helper cells processes said pre-protein produced by said bacterial producer cells.

9. The method of claim 8, wherein said helper protein is a protease.

10. The method of claim 1, wherein said molecule of interest produced by said bacterial producer cells is an antibody.

11. The method of claim 1 or 10, wherein said helper protein expressed by said bacterial helper cells binds said antibody produced by said bacterial producer cells.

12. The method of claim 11, wherein said helper protein is protein A, G or L.

13. The method of any one of the preceding claims, wherein, after separate pre-culturing, said bacterial producer cells and said bacterial helper cells are mixed in a pre-defined ratio for being co-cultured.

14. Use of co-cultured bacterial producer cells and bacterial helper cells, wherein said bacterial producer cells and/or bacterial helper cells are growth and production decoupled, wherein said bacterial producer cells are genetically-engineered to produce said molecule of interest, and said bacterial helper cells are genetically-engineered to produce a helper protein aiding in the production of said molecule of interest, for the production of a molecule of interest.

15. A bacterial consortium comprising bacterial producer cells which are genetically-engineered to produce a molecule of interest and bacterial helper cells which are genetically-engineered to produce a protein aiding in the production of said molecule of interest, wherein each of said bacterial producer cells and bacterial helper cells is equipped such that growth and production can be decoupled.
